# EUROPÄISCHE PATENTSCHRIFT

(11) **EP 1 794 110 B1**
(45) Veröffentlichungstag und Bekanntmachung des Hinweises auf die Patenterteilung: **05.01.2011**
(21) Anmeldenummer: 05784061.3
(22) Anmeldetag: 22.09.2005
(51) Int. Cl.: C07C 45/28, C07C 49/00, C01B 21/22, B01D 53/14

(54) **VERFAHREN ZUR REINIGUNG UND AUFKONZENTRIERUNG VON DISTICKSTOFFMONOXID**
METHOD FOR PURIFYING AND CONCENTRATING DINITROGEN MONOXIDE
PROCEDE POUR PURIFIER ET CONCENTRER DU MONOXYDE DE DIAZOTE

(30) Priorität: 23.09.2004 DE 102004046167
(43) Veröffentlichungstag der Anmeldung: 13.06.2007
(73) Patentinhaber: BASF SE, 67056 Ludwigshafen (DE)
(72) Erfinder: TELES, Joaquim Henrique, 67166 Otterstadt (DE); RÖSSLER, Beatrice, 67098 Bad Dürkheim (DE); GENGER, Thomas, 67245 Lambsheim (DE); GLASS, Andreas, 67245 Lambsheim (DE); BAUMANN, Dieter, 69190 Walldorf (DE); LÖNING, Jan-Martin, 26831 Dollart (DE)
(86) Internationale Anmeldenummer: PCT/EP2005/010267
(87) Internationale Veröffentlichungsnummer: WO 2006/032502

(56) Entgegenhaltungen:
- WO-A-2005/030689
- WO-A-2005/030690
- DE-A1- 3 708 469

## Beschreibung

Die vorliegende Erfindung betrifft ein Verfahren zur Reinigung eines Gasgemisches enthaltend Distickstoffmonoxid sowie die Verwendung eines derartig gereinigten Gasgemisches als Oxidationsmittel für Olefine. In einer weiteren Ausführungsform betrifft die vorliegende Erfindung auch ein Verfahren zur Herstellung von Ketonen umfassend die Oxidation mindestens eines Olefins mit einem erfindungsgemäß gereinigten Gasgemisch enthaltend Distickstoffmonoxid.

Aus dem Stand der Technik sind verschiedene Herstellungsverfahren für Distickstoffinonoxid bekannt. Es ist ebenfalls bekannt, dass Distickstoffmonoxid beispielsweise als Oxidationsmittel für Olefine eingesetzt werden kann.

So offenbart die WO 98/25698 ein Verfahren zur Herstellung von Distickstoffmonoxid durch katalytische Partialoxidation von NH₃ mit Sauerstoff. Dabei wird gemäß der WO 98/25698 ein Katalysator aus Manganoxid, Bismutoxid und Aluminiumoxid eingesetzt, der mit hoher Selektivität zu Distickstoffmonoxid führt. Ein ähnliches Katalysatorsystem wird auch in einer wissenschaftlichen Arbeit näher beschrieben (Noskov et al., Chem. Eng. J. 91 (2003) 235-242). Gemäß der US 5,849,257 wird ebenfalls ein Verfahren zur Herstellung von Distickstoffmonoxid durch Oxidation von Ammoniak offenbart. Dabei findet die Oxidation in Gegenwart eines Kupfer-Manganoxid Katalysators statt.

Gemäß dem in der WO 00/01654 offenbarten Verfahren wird Distickstoffmonoxid hergestellt, indem ein Gasstrom enthaltend NOₓ und Ammoniak reduziert wird.

Die Oxidation einer olefinischen Verbindung zu einem Aldehyd oder einem Keton mittels Distickstoffmonoxid ist beispielsweise beschrieben in der GB 649,680 oder der dazu äquivalenten US 2,636,898. In beiden Schriften wird ganz allgemein offenbart, dass die Oxidation prinzipiell in Anwesenheit eines geeigneten Oxidationskatalysators erfolgen kann.

In den neueren wissenschaftlichen Artikeln von G. L. Panov et al., "Non-Catalytic Liquid Phase Oxidation of Alkenes with Nitrous Oxide. 1. Oxidation of Cyclohexene to Cyclohexanone", React. Kinet. Catal. Lett. Vol. 76, No. 2 (2002) S. 401-405, und K. A. Dubkov et al., "Non-Catalytic Liquid Phase Oxidation of Alkenes with Nitrous Oxide. 2. Oxidation of Cyclopentene to Cyclopentanone", React. Kinet. Catal. Lett. Vol. 77, No. 1 (2002) S. 197-205 werden ebenfalls Oxidationen von olefinischen Verbindungen mit Distickstoffmonoxid beschrieben. Auch ein wissenschaftlicher Artikel "Liquid Phase Oxidation of Alkenes with Nitrous Oxide to Carbonyl Compounds" von E. V. Starokon et al. in Adv. Synth. Catal. 2004, 346, 268 - 274 beinhaltet eine mechanistische Studie der Oxidation von Alkenen mit Distickstoffmonoxid in flüssiger Phase.

Die Synthese von Carbonylverbindungen aus Alkenen mit Distickstoffmonoxid wird auch in verschiedenen internationalen Patentanmeldungen beschrieben. So offenbart die WO 03/078370 ein Verfahren zur Herstellung von Carbonylverbindungen aus aliphatischen Alkenen mit Distickstoffmonoxid. Die Umsetzung wird bei Temperaturen im Bereich von 20 bis 350°C und Drücken von 0,01 bis 100 atm durchgeführt. Die WO 03/078374 offenbart ein entsprechendes Verfahren zur Herstellung von Cyclohexanon. Gemäß der WO 03/078372 werden cyclische Ketone mit 4 bis 5 C-Atomen hergestellt. Gemäß der WO 03/078375 werden unter diesen Verfahrensbedingungen cyclische Ketone aus cyclischen Alkenen mit 7 bis 20 C-Atomen hergestellt. WO03/078371 offenbart ein Verfahren zur Herstellung substituierter Ketone aus substituierten Alkenen. WO 04/000777 offenbart ein Verfahren zur Umsetzung von Di- und Polyalkenen mit Distickstoffmonoxid zu den entsprechenden Carbonylverbindungen. Die Reinigung von Distickstoffmonoxid wird in diesen Schriften nicht erwähnt.

Es ist ebenfalls bekannt, dass Abgasströme enthaltend Distickstoffmonoxid für weitere Umsetzungen eingesetzt werden können. Distickstoffmonoxid fällt als unerwünschtes Nebenprodukt bei verschiedenen chemischen Prozessen an, insbesondere bei Oxidationen mit Salpetersäure und dort ganz besonders bei der Oxidation von Cyclohexanon und/oder Cyclohexanol zu Adipinsäure. Andere Beispiele für Verfahren, bei denen Distickstoffmonoxid als unerwünschtes Nebenprodukt anfällt, sind die Oxidation von Cyclododecanon und/oder Cyclododecanol mit Salpetersäure zu Dodecandicarbonsäure und die Partialoxidation von NH₃ zu NO.

So offenbaren die DE 103 44 595.1, die DE 103 44 594.3 und die DE 103 19 489.4 Verfahren zur Oxidation von Olefinen mit Distickstoffmonoxid, nämlich die Oxidation von Cyclododecatrien, von Cyclododecen und von Cyclopenten. Alle drei Anmeldungen offenbaren, dass neben anderen Distickstoffmonoxidquellen auch Abgasströme eingesetzt werden können, die beispielsweise durch destillative Methoden aufgereinigt werden können, bevor sie als Oxidationsmittel eingesetzt werden.

Sowohl bei der Herstellung von Distickstoffmonoxid als auch bei der Verwendung von Abgasströmen fällt N₂O zunächst als verdünntes gasförmiges Gemisch mit anderen Komponenten an. Diese Komponenten lassen sich unterteilen in solche, die für spezielle Anwendungen störend wirken, und solche, die sich inert verhalten. Für den Einsatz als Oxidationsmittel sind als solche störend wirkenden Gase unter anderem NOₓ oder beispielsweise Sauerstoff zu nennen. Der Begriff "NOₓ", wie er im Rahmen der vorliegenden Erfindung verstanden wird, bezeichnet sämtliche Verbindungen NₐO_{b}, wobei a 1 oder 2 ist und b eine Zahl von 1 bis 6, außer N₂O. Statt dem Begriff "NOₓ" wird im Rahmen der vorliegenden Erfindung auch der Begriff "Stickoxide" verwendet. Als störende Nebenkomponenten sind auch NH₃ und organischen Säuren zu nennen.

Für spezielle Anwendungen ist es nötig, das eingesetzte Distickstoffmonoxid vor der Umsetzung zu reinigen. Beispielsweise für die Verwendung von Distickstoffmonoxid als Oxidationsmittel ist es nötig, störende Nebenkomponenten wie Sauerstoff oder Stickoxide NOₓ abzutrennen.

Verfahren zur Abtrennung von NOₓ sind grundsätzlich aus dem Stand der Technik bekannt. Eine Übersicht gibt beispielsweise M. Thiemann et. al in Ullmann's Encyclopedia, 6th Edition, 2000, Electronic Edition, Kapitel "Nitric Acid, Nitrous Acid, and Nitrogen Oxides", Abschnitt 1.4.2.3.

Die Anmeldung WO 00/73202 beschreibt eine Methode, wie man NOₓ und O₂ aus einem N₂O-haltigen Gasstrom entfernen kann. Das NOₓ wird durch katalytische Reduktion mit NH₃ entfernt und Sauerstoff durch katalytische Reduktion mit Wasserstoff oder anderen Reduktionsmitteln. Diese Methode hat aber den Nachteil, dass das Produkt mit NH₃ kontaminiert wird. Eine starke Abreicherung von Sauerstoff (z.B. auf über 90% der ursprüngliche Menge) ist nur möglich, wenn eine Verlust an N₂O in Kauf genommen wird (von 3 bis 5% der ursprünglich enthaltenen Menge).

Für spezielle Anwendungen kann es notwendig sein, auch die inerten Verbindungen abzutrennen, da sie die gewünschte Umsetzung mit N₂O durch Verdünnung verlangsamen können. Der Begriff "Inertgas", wie er im Rahmen der vorliegenden Erfindung verwendet wird, bezeichnet ein Gas, das sich hinsichtlich der Umsetzung von N₂O mit einem Olefin inert verhält, also unter den Bedingungen der Umsetzung von Olefinen mit N₂O weder mit den Olefinen noch mit N₂O reagieren. Als Inertgase sind beispielsweise Stickstoff, Kohlendioxid, Kohlenmonoxid, Wasserstoff, Argon, Methan, Ethan und Propan zu nennen. Die Inertgase senken aber die Raum-Zeit-Ausbeute, so dass eine Abreicherung ebenfalls vorteilhaft ist.

In der DE 27 32 267 A1 wird beispielsweise ein Verfahren zur Reinigung von Distickstoffmonoxid offenbart, wobei zunächst Stickstoffoxid, Stickstoffdioxid, Kohlenstoffdioxid und Wasser abgetrennt werden und anschließend das Gasgemisch durch Kompression auf 40 bis 300 bar und Kühlung aus 0 bis -88°C verflüssigt wird. Aus diesem verflüssigten Gasgemisch wird dann Distickstoffmonoxid abgetrennt. Diese Methode erreicht zwar eine Reinigung und Aufkonzentrierung des N₂O, ist aber aufgrund des geforderten hohen Drucks (60 bar), der tiefen Temperaturen (-85°C) und den damit verbundenen hohen Investitionen, wirtschaftlich unattraktiv.

In der US 4,177,645 wird ein Verfahren zur Abtrennung von Distickstoffmonoxid aus Abgasströmen offenbart, das ebenfalls eine Vorreinigung und eine Tieftemperaturdestillation umfasst. Die Anmeldung EP 1 076 217 A1 beschreibt ebenfalls eine Methode zur Entfernung von leichtsiedenden Verunreinigungen aus N₂O durch Tieftemperaturdestillation.

Auch die US 6,505,482, die US 6,370,911 und die US 6,387,161 offenbaren Verfahren zur Reinigung von Distickstoffmonoxid, bei dem jeweils eine Tieftemperaturdestillation in einer speziellen Anlage durchgeführt wird.

Eine Tieftemperaturdestillation erfordert durch die hohen Drücke und tiefen Temperaturen jedoch apparativ einen hohen Aufwand, der die Reinigung des Distickstoffmonoxids mit einem derartigen Verfahren aufwändig und kostenintensiv macht. Besonders störend ist hierbei die Tatsache, dass bei Normaldruck der Schmelzpunkt von N₂O nur 3K unterhalb des Siedepunkts liegt. Daher müssen hohe Drücke angewandt werden.

DE 20 40 219 offenbart ein Herstellungsverfahren für Distickstoffmonoxid, wobei das erhaltene Distickstoffmonoxid nach der Synthese konzentriert und gereinigt wird. Dabei wird gemäß der DE 20 40 219 zunächst Distickstoffmonoxid durch Oxidation von Ammoniak hergestellt. Das hergestellte Distickstoffmonoxid wird gereinigt, indem die oxidierten Gase separiert und durch Absorption unter hohem Druck, der eine Desorption unter vermindertem Druck folgt, konzentriert. Nebenkomponenten werden beispielsweise durch Behandlung mit einer Alkalilösung entfernt. Als Lösungsmittel für die Absorption des Gasgemisches wird gemäß der DE 20 40 219 Wasser verwendet.

Mit dem in der DE 20 40 219 offenbarten Verfahren ist eine Trennung der verschiedenen Stickoxide möglich, das Verfahren erfordert jedoch auf Grund der geringen Löslichkeit von N₂O in Wasser den Einsatz von großen Lösungsmittelmengen und/oder hoher Drücke für die Absorption. Dies führt dazu, dass auch die verwendeten Anlagen auf große Volumen ausgelegt sein müssen. Dies macht das Verfahren insgesamt unwirtschaftlich.

Ausgehend von diesem Stand der Technik lag eine Aufgabe der vorliegenden Erfindung darin, ein Verfahren bereitzustellen, mit dem Distickstoffmonoxid haltige Ströme effektiv und kostengünstig gereinigt und aufkonzentriert werden können, d.h. gleichzeitig störende und inerte Komponenten abgetrennt werden können. Derart aufgereinigtes Distickstoffmonoxid wird insbesondere als Oxidationsmittel benötigt.

Erfindungsgemäß wird diese Aufgabe gelöst durch ein Verfahren zur Reinigung eines Gasgemisches enthaltend Distickstoffmonoxid, mindestens umfassend die folgenden Schritte:
- A1: Absorprtion des Gasgemisches in einem organischen Lösungsmittel
- A2: Desorption des Gasgemisches aus dem beladenen organischen Lösungsmittel
- B: Einstellen des Gehalts an Stickoxiden NOₓ in dem Gasgemisch auf höchstens 0,5 Vol.-%, bezogen auf das Gesamtvolumen des Gasgemisches.

Das erfindungsgemäße Verfahren hat unter anderem den Vorteil, dass neben den störenden Komponenten auch die inerten Komponenten abgetrennt werden. Das erfindungsgemäß gereinigte Distickstoffmonoxid wird also gleichzeitig aufkonzentriert. Derart gereinigtes Distickstoffmonoxid kann insbesondere in flüssiger Form vorteilhaft als Oxidationsmittel eingesetzt werden, da keine weiteren inerten Verbindungen das Reaktionsvolumen vergrößern und die Reaktion verlangsamen.

Dabei kann das eingesetzte Gasgemisch enthaltend Distickstoffmonoxid grundsätzlich aus jeder beliebigen Quelle stammen.

Der Begriff "Gasgemisch", wie er im Rahmen der vorliegenden Erfindung verwendet wird, bezeichnet ein Gemisch aus zwei oder mehr Verbindungen, die sich bei Umgebungsdruck und Umgebungstemperatur im gasförmigen Zustand befinden. Bei veränderter Temperatur oder verändertem Druck kann das Gasgemisch auch in einem anderen Aggregatzustand vorliegen, beispielsweise flüssig, und wird im Rahmen der vorliegenden Erfindung weiter als Gasgemisch bezeichnet.

Wird ein Gasgemisch eingesetzt, so ist dessen Gehalt an Distickstoffmonoxid im Wesentlichen beliebig, solange gewährleistet ist, dass die erfindungsgemäße Reinigung möglich ist.

Die N₂O-haltigen Gasgemische, die für dieses Verfahren eingesetzt werden, haben in der Regel einen N₂O-Gehal zwischen 2 und 80 Vol.-% N₂O. Es enthält ferner beispielsweise 2 bis 21 Vol.-% O₂ und bis zu 30 Vol.-% NOₓ als unerwünschte Komponenten. Ferner kann es noch in wechselnden Mengen N₂, H₂, CO₂, CO, H₂O, NH₃ enthalten, in Spuren können auch noch Salpetersäure und organische Verbindungen enthalten sein.

Im Rahmen der vorliegenden Erfindung wird die Zusammensetzung der Gasgemische oder der verflüssigten Gasgemische in Vol.-% angegeben. Dabei beziehen sich die Angaben auf die Zusammensetzung der Gasgemische bei Umgebungsdruck und Umgebungstemperatur.

Gemäß einer bevorzugten Ausführungsform des erfindungsgemäßen Verfahrens wird ein mindestens 5 Vol.-% Distickstoffmonoxid enthaltendes Gasgemisch eingesetzt, wobei wiederum bevorzugt Gemische mit einem Distickstoffmonoxid-Gehalt im Bereich von 6 bis 80 Vol.-%, weiter bevorzugt im Bereich von 7 bis 60 Vol.-% und insbesondere bevorzugt im Bereich von 8 bis 50 Vol.-% eingesetzt werden.

Grundsätzlich kann die Zusammensetzung der Gemische im Rahmen der vorliegenden Erfindung auf jede dem Fachmann bekannte Weise bestimmt werden. Die Zusammensetzung der Gasgemische wird im Rahmen der vorliegenden Erfindung vorzugsweise gaschromatographisch bestimmt. Sie kann jedoch auch mittels UV-Spektroskopie, IR-Spektroskopie oder nasschemisch bestimmt werden.

Gemäß einer bevorzugten Ausführungsform der vorliegenden Erfindung ist das Gasgemisch enthaltend Distickstoffmonoxid mindestens ein Distickstoffmonoxid enthaltendes Abgas eines chemischen Verfahrens. Im Rahmen der vorliegenden Erfindung sind auch Ausführungsformen umfasst, bei denen mindestens zwei Stickstoffmonoxid enthaltende Abgase einer einzigen Anlage als Gasgemisch enthaltend Distickstoffmonoxid dienen. Ebenso sind Ausführungsformen umfasst, bei denen mindestens ein Distickstoffmonoxid enthaltendes Abgas einer Anlage und mindestens ein weiteres Distickstoffmonoxid enthaltendes Abgas mindestens einer weiteren Anlage als Gasgemisch enthaltend Distickstoffmonoxid dienen.

Demgemäß betrifft die vorliegende Erfindung auch ein wie oben beschriebenes Verfahren, das dadurch gekennzeichnet ist, dass das Gasgemisch enthaltend Distickstoffmonoxid mindestens ein Distickstoffmonoxid enthaltendes Abgas mindestens eines industriellen Verfahrens ist.

Der Begriff "Gasgemisch enthaltend Distickstoffmonoxid" bezeichnet im Rahmen der vorliegende Erfindung sowohl Ausführungsformen, in denen das genannte Abgas in unmodifizierter Form dem erfindungsgemäßen Reinigungsverfahren unterworfen wird, als auch Ausführungsformen, in denen mindestens eines der genannten Abgase einer Modifikation unterworfen wird.

Der Begriff "Modifikation", wie er in diesem Zusammenhang im Rahmen der vorliegenden Erfindung verwendet wird, bezeichnet jedes geeignete Verfahren, mit dem die chemische Zusammensetzung eines Gasgemisches verändert wird. Demgemäß umfasst der Begriff "Modifikation" unter anderem Ausführungsformen, in denen ein Distickstoffmonoxid enthaltendes Abgas bezüglich des Distickstoffmonoxidgehaltes gemäß mindestens eines geeigneten Verfahrens aufkonzentriert wird. Vorzugsweise wird das Abgas keiner Modifikation unterworfen.

Gemäß einer weiteren Ausführungsform kann die chemische Zusammensetzung eines Abgases auch durch Zusatz von reinem Distickstoffmonoxid zu dem Abgas verändert werden.

Das eingesetzte Gasgemisch enthaltend N₂O kann beispielsweise ein Abgas aus einem industriellen Verfahren sein. Vorzugsweise stammt es aus einem Abgas einer Anlage zur Herstellung von Carbonsäuren durch Oxidation von Alkoholen oder Ketonen mit Salpetersäure, wie z.B. aus einer Adipinsäure- oder Dodecandicarbonsäure-Anlage, aus dem Abgas einer Salpetersäure-Anlage die die obigen Abgasströme als Edukt einsetzt, aus dem Abgas einer Anlage zur Partialoxidation von NH₃ oder aus dem Abgas einer Anlage die die darin erzeugten Gasgemische einsetzt, wie z.B. einer Hydroxylamin-Anlage.

Erfindungsgemäß kann auch ein Gemisch verschiedener Abgase eingesetzt werden.

Gemäß einer weiter bevorzugten Ausführungsform der vorliegenden Erfindung stammt das mindestens eine Distickstoffmonoxid enthaltende Abgas aus einer Adipinsäureanlage, einer Dodecandisäureanlage, einer Hydroxylaminanlage und/oder einer Salpetersäureanlage, wobei letztere wiederum bevorzugt mit mindestens einem Abgas einer Adipinsäureanlage, einer Dodecandisäureanlage oder einer Hydroxylaminanlage betrieben wird.

Gemäß einer bevorzugten Ausführungsform wird der Abgasstrom einer Adipinsäureanlage eingesetzt, bei der durch Oxidation von Cyclohexanol/Cyclohexanon-Gemischen mit Salpetersäure pro Mol gebildeter Adipinsäure im Allgemeinen 0,8 bis 1,0 mol N₂O gebildet werden. Wie beispielsweise in A. K. Uriarte et al., Stud. Surf. Sci. Catal. 130 (2000) S. 743-748 beschrieben, enthalten die Abgase von Adipinsäureanlagen in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Bei der obenstehend erwähnten Dodecandisäureanlage handelt es sich um den im Wesentlichen identischen Anlagentyp.

Eine beispielsweise typische Zusammensetzung eines Abgases einer Adipinsäureanlage oder einer Dodecandisäureanlage ist in der folgenden Tabelle wiedergegeben:

| Komponente | Konzentrationen / Gew.-% |
|---|---|
| NOₓ | 19 - 25 |
| N₂O | 20-28 |
| N₂ | 30-40 |
| O₂ | 7-10 |
| CO₂ | 2-3 |
| H₂O | ∼ 7 |

Der Abgasstrom einer Adipinsäureanlage oder einer Dodecandisäureanlage kann direkt in dem erfindungsgemäßen Verfahren eingesetzt werden.

Gemäß einer ebenfalls bevorzugten Ausführungsform wird der Abgasstrom einer Salpetersäureanlage eingesetzt, die ganz oder teilweise mit Distickstoffmonoxid und Stickoxide enthaltenden Abgasen aus anderen Verfahren gespeist wird. In derartigen Salpetersäureanlagen werden Stickoxide adsorbiert und zum größten Teil zu Salpetersäure umgesetzt, während Distickstoffmonoxid nicht umgesetzt wird. Beispielsweise kann eine derartige Salpetersäureanlage durch Stickoxide, die durch gezielte Verbrennung von Ammoniak hergestellt werden, und durch Abgase einer Adipinsäureanlage und/oder durch Abgase einer Dodecandisäureanlage gespeist werden. Ebenso ist es möglich, eine derartige Salpetersäureanlage allein durch Abgase einer Adipinsäureanlage und/oder durch Abgase einer Dodecandisäureanlage zu speisen.

Die Abgase von derartigen Salpetersäureanlagen enthalten grundsätzlich in unterschiedlichen Konzentrationen noch weitere Bestandteile wie unter anderem Stickstoff, Sauerstoff, Kohlendioxid, Kohlenmonoxid, Stickoxide, Wasser und flüchtige organische Verbindungen.

Eine beispielsweise typische Zusammensetzung eines Abgases einer derartigen Salpetersäureanlage ist in der folgenden T. belle wiedergegeben:

| Komponente | Konzentrationen / Gew.-% |
|---|---|
| NOₓ | < 0,1 |
| N₂O | 8 - 36 |
| N₂ | 57 - 86 |
| O₂ | 3-9 |
| CO₂ | 1-4 |
| H₂O | ∼ 0,6 |

Der Abgasstrom einer Salpetersäureanlage kann direkt in dem erfindungsgemäßen Verfahren eingesetzt werden.

Gemäß einer ebenfalls bevorzugten Ausführungsform des erfindungsgemäßen Verfahrens wird der Abgasstrom einer Hydroxylaminanlage eingesetzt, bei der beispielsweise zunächst Ammoniak mit Luft oder Sauerstoff zu NO oxidiert wird, wobei kleine Mengen an Distickstoffmonoxid als Nebenprodukt gebildet werden. Das NO wird anschließend mit Wasserstoff zu Hydroxylamin hydriert. Nachdem Distickstoffmonoxid unter den Hydrierbedingungen inert ist, reichert es sich im Wasserstoffkreis an. In bevorzugten Verfahrensführungen enthält der Purge-Strom einer Hydroxylaminanlage Distickstoffmonoxid im Bereich von 9 bis 13 Vol.-% in Wasserstoff. Dieser Purge-Strom kann als solcher zur erfindungsgemäßen Reinigung eingesetzt werden. Ebenso ist es möglich, diesen Strom bezüglich des Distickstoffmonoxidgehaltes, wie oben beschrieben, geeignet aufzukonzentrieren.

Demgemäß betrifft die vorliegende Erfindung auch ein wie oben beschriebenes Verfahren, das dadurch gekennzeichnet ist, dass das Gasgemisch enthaltend Distickstoffmonoxid das Abgas einer Adipinsäureanlage und/oder einer Dodecandisäureanlage und/oder einer Hydroxylaminanlage und/oder einer mit dem Abgas einer Adipinsäureanlage und/oder einer Dodecandisäureanlage und/oder einer Hydroxylaminanlage betriebenen Salpetersäureanlage ist.

Ebenso kann im Rahmen des erfindungsgemäßen Verfahrens Distickstoffmonoxid zum Einsatz im Verfahren gezielt hergestellt werden. Bevorzugt wird dabei unter anderem die Herstellung über die thermische Zersetzung von NR₄NO₃, wie dies beispielsweise in der US 3,656,899 beschrieben ist. Ebenfalls bevorzugt wird weiter die Herstellung über die katalytische Oxidation von Ammoniak, wie dies beispielsweise in der US 5,849,257 oder in der WO 98/25698 beschrieben ist.

Das gewünschte Produkt enthält mindestens 50 Vol.-% N₂O, besonders bevorzugt mindestens 60 Vol.-% N₂O und ganz besonders bevorzugt mindestens 75 Vol.-% N₂O. Gleichzeitig enthält das Produkt weniger als 1 Vol.-% O₂, insbesondere weniger als 0,5 Vol.-% O₂, weniger als 0,5 Vol.-% NOₓ und weniger als 1 Vol.-% NH₃.

Die N₂O-Konzentrierung erfolgt erfindungsgemäß durch selektive Absorption von N₂O aus dem Rohgasgemisch in einem geeigneten organischen Lösungsmittel und anschließender Desorption von N₂O aus dem beladenen Lösungsmittel gemäß Schritt A1 und A2.

Geeignete Lösungsmittel für die Absorption gemäß Schritt A1 sind solche, die für N₂O eine bessere Löslichkeit aufweisen, als für die unerwünschten Komponenten des eintretenden Eduktgases.

Als organische Lösungsmittel können erfindungsgemäß alle Lösungsmittel eingesetzt werden, bei denen das Verhältnis zwischen N₂O-Löslichkeit (in mol/mol Lösungsmittel) und der Löslichkeit der unerwünschten Nebenkomponenten unter den im Absorber herrschenden Bedingungen (dieses Verhältnis wird im folgenden □ genannt) mindestens 5 beträgt. Dies Verhältnis kann für jede einzelne im Gasgemisch enthaltene Komponente bestimmt werden. Bevorzugte organische Lösungsmittel weisen beispielsweise bei 30 °C einen Wert □_{O2} von 6 bis 30, bevorzugt von 9 bis 25 und einen Wert □_{N2} von größer 10, bevorzugt von größer als 20, insbesondere von größer als 30 auf.

Beispiele für geeignete Lösungsmittel sind beispielsweise aliphatische Kohlenwasserstoffe, bevorzugt mit mindestens 5 C-Atomen, weiter bevorzugt mit mindestens 8 C-Atomen, substituierte oder unsubstituierte aromatische Kohlenwasserstoffe, Ester, Ether, Amide, Lactone, Lactame, Nitrile, Alkylhalogenide, Olefine oder Mischungen dieser Lösungsmittel.

Ganz besonders bevorzugt sind erfindungsgemäß Lösungsmittel, die einen Siedepunkt bei Normaldruck von mindestens 100°C aufweisen, da dadurch die Lösungsmittelverluste sowohl im Abgasstrom des Absorbers wie auch des Desorbers reduziert werden.

Darüber hinaus weisen erfindungsgemäß geeignete Lösungsmittel gleichzeitig eine gute Löslichkeit für Distickstoffmonoxid auf. Die Löslichkeit wird über das Verhältnis zwischen dem Partialdruck von N₂O in der Gasphase und dem Gewichtsanteil von N₂O in der Flüssigphase (Henry-Koeffizient, H_{N2O}) angegeben, d.h. ein kleiner Wert bedeutet eine hohe Löslichkeit von Distickstoffmonoxid im Lösungsmittel. Vorzugsweise ist dies Verhältnis für ein erfindungsgemäß eingesetztes organisches Lösungsmittel bei 30°C kleiner als 1000, weiter bevorzugt kleiner als 750, besonders bevorzugt kleiner als 500, insbesondere kleiner als 260.

Geeignete Lösungsmittel sind unter anderem N-Methylpyrrolidon, Dimethylformamid, Dimethylsulfoxid, Propylencarbonat, Sulfolan, N,N-Dimethylacetamid oder Cyclopentan. Besonders bevorzugt sind im Rahmen der vorliegenden Erfindung beispielsweise Toluol, Nitrobenzol, 1,2-Dichlorbenzol, Tetradecan, beispielsweise ein technisches Gemisch aus gesättigten Kohlenwasserstoffen mit überwiegend 14 Kohlenstoffatomen, und Phthalsäuredimethylester.

Daher betrifft die vorliegende Erfindung in einer Ausführungsform ein Verfahren zur Reinigung eines Gasgemisches umfassend Distickstoffmonoxid wie oben beschrieben, wobei das im Schritt A1 eingesetzte organische Lösungsmittel ausgewählt ist aus der Gruppe bestehend aus Toluol, Nitrobenzol, 1,2-Dichlorbenzol, Tetradecan und Phthalsäuredimethylester.

Die Absorption gemäß Schritt A1 des erfindungsgemäßen Verfahrens kann grundsätzlich nach allen dem Fachmann bekannten Verfahren erfolgen. Insbesondere kann die Absorption von N₂O im Lösungsmittel durch Erhöhung des Drucks des Eduktgases oder durch Absenkung der Temperatur des Lösungsmittels oder durch eine Kombination der genannten Maßnahmen herbeigeführt werden.

Die Absorption erfolgt erfindungsgemäß in Einrichtungen (Absorber) in denen eine Gas-Flüssig-Phasengrenzfläche erzeugt wird, über die ein Stoff- und Wärmeübergang zwischen den Phasen ermöglicht wird, und die bei Bedarf mit internen oder externen Einrichtungen zur Wärmezufuhr und/oder Wärmeabfuhr versehen sind.

Die Führung der Phasen im Absorber kann im Gleichstrom, im Gegenstrom oder einer Kombination der genannten erfolgen.

Die Absorption kann erfindungsgemäß ein- oder mehrstufig durchgeführt werden.

Mögliche Ausführungsformen des Absorbers sind Kolonnen mit Böden, beispielsweise Glockenböden oder Siebböden, Kolonnen mit strukturierten Einbauten, wie beispielsweise Packungen, Kolonnen mit unstrukturierten Einbauten, wie beispielsweise Füllkörpern, oder Apparate in denen die Flüssigphase dispergiert vorliegt, beispielsweise durch Versprühen in Düsen, oder eine Kombination der genannten.

Die Desorption von N₂O aus dem beladenen Lösungsmittel gemäß Schritt A2 des erfindungsgemäßen Verfahrens kann durch Druckabsenkung über dem Lösungsmittel, Temperaturerhöhung des Lösungsmittels oder durch Strippung mit Lösungsmitteldampf oder einer Kombination der genannten herbeigeführt werden.

Die Anforderungen an die Einrichtungen (Desorber) zur Desorption von N₂O aus dem beladenen Lösungsmittel, sowie die Führung der Phasen sind analog zu denen des Absorbers, d.h. geeignet sind Einrichtungen, in denen eine Gas-Flüssig-Phasengrenzfläche erzeugt wird, über die ein Stoff- und Wärmeübergang zwischen den Phasen ermöglicht wird, und die bei Bedarf mit internen oder externen Einrichtungen zur Wärmezufuhr und/oder Wärmeabfuhr versehen sind.

Die Desorption kann erfindungsgemäß ein- oder mehrstufig durchgeführt werden.

Mögliche Ausführungsformen des Desorbers sind ein einfacher (Entspannungs-) Behälter und Kolonnen.

Eine Ausführungsform der vorliegenden Erfindung, in der die Absorption und Desorption apparativ vereint sind, ist die Trennwandkolonne. Dabei wird die Absorption und Desorption durch Temperaturwechsel mehrstufig im Gegenstrom betrieben kombiniert mit einer Strippung mit Lösungsmitteldampf.

In einer Ausführungsform betrifft die vorliegende Erfindung daher ein Verfahren wie oben beschrieben, wobei die Schritte A1 und A2 in einer Trennwandkolonne durchgeführt werden.

Im Sinne einer besonders bevorzugten Ausführungsform der Erfindung wird gemäß Schritt A1 zunächst das Gasgemisch enthaltend N₂O unter erhöhtem Druck p_{Abso} in einer im Gegenstrom betriebenen Absorptionskolonne mit Füllkörperschüttung absorbiert und gemäß Schritt A2 in einen Behälter überführt, in dem das mit N₂O beladene Lösungsmittel auf einen niedrigeren Druck p_{Deso} < p_{Abso} entspannt wird. Der Prozess wird vorzugsweise nahezu isotherm betrieben mit einer Temperaturdifferenz zwischen Absorptions- und Desorptionstemperatur von maximal 20K, vorzugsweise maximal 15K, insbesondere maximal 10 K. Der Absorptionsdruck beträgt hierbei 1 bis 100 bar, bevorzugt 5 bis 65 bar, insbesondere 10 bis 40 bar und der Desorptionsdruck 0,1 bis 2 bar, vorzugsweise 0,5 bis 1,5 bar, besonders bevorzugt 1,0 bis 1,2 bar.

Eine weitere Ausführungsform der Erfindung betrifft ein Verfahren, bei dem zwei oder mehr Distickstoffmonoxid-Konzentrierungsstufen umfassend Schritte A1 und A2 nacheinander durchgeführt werden.

Hierbei dient das in der Stufe i-1 desorbierte Gas, als Eduktgas der darauf folgenden Stufe i. Im Sinne der Erfindung besonders bevorzugt ist eine zweistufige Ausführungsform.

Das Lösungsmittel kann hierbei für alle Stufen identisch sein. Alternativ können in unterschiedlichen Stufen verschiedene Lösungsmittel eingesetzt werden.

Daher betrifft die vorliegende Erfindung in einer weiteren Ausführungsform ein Verfahren wie oben beschrieben, wobei das Verfahren mehrere Schritte A1 und A2 umfasst.

Das erfindungsgemäße Verfahren umfasst weiterhin einen Schritt B, wobei der Gehalt an Stickoxiden im Gasgemisch auf höchstens 0,5 Vol.-% bezogen auf das Gesamtvolumen des Gasgemisches eingestellt wird.

Dabei kann im Rahmen der vorliegenden Erfindung Schritt B vor oder nach den Schritten A1 und A2 durchgeführt werden. Daher betrifft die vorliegende Erfindung in einer Ausführungsform ein Verfahren wir oben beschrieben, wobei die Schritte A1 und A2 vor dem Schritt B ausgeführt werden. In einer alternativen Ausführungsform betrifft die vorliegende Erfindung ein Verfahren wir oben beschrieben, wobei der Schritt B vor den Schritte A1 und A2 ausgeführt wird

Ebenso ist es im Rahmen der vorliegenden Erfindung möglich, dass das Verfahren mehrere Schritte B umfasst. Damit kann beispielsweise auch ein Schritt B vor den Schritten A1 und A2 durchgeführt werden und ein weiterer Schritt B nach den Schritten A1 und A2.

Grundsätzlich kommen für den Schritt B des erfindungsgemäßen Verfahrens alle geeigneten Verfahren zur Abtrennung von NOₓ in Frage. Geeignet sind beispielsweise die katalytische Reduktion mit Kohlenwasserstoffen oder Ammoniak, die katalytische Zersetzung an geeigneten Katalysatoren, Absorption in stark oxidierenden Lösungen sowie die Absorption in sauren oder alkalischen Lösungen.

Geeignete oxidierende Lösungen im Rahmen der vorliegenden Erfindung sind beispielsweise Lösungen von Wasserstoffperoxid. Als stark saure Lösungen sind erfindungsgemäß beispielsweise Lösungen enthaltend Salpetersäure oder Schwefelsäure geeignet. Als alkalische Lösungen eignen sich erfindungsgemäß beispielsweise Lösungen enthaltend Hydroxide oder Carbonate, beispielsweise Natriumhydroxid oder Natriumcarbonat. Als Flüssigkeiten für diese Wäsche eignen sich neben den bereits genannten insbesondere diejenige, die dem Fachmann zur Entfernung von NOₓ aus Abgasen geläufig sind. Als Waschlösungen oder - suspensionen sind beispielsweise geeignet wässrige Lösungen oder Suspensionen enthaltend Magnesiumcarbonat, Magnesiumhydroxid, Lösungen von Vanadium in salpetriger Säure, Ammoniumsulfid und Ammoniumbisulfid, Kalkwasser, Ammoniak, Wasserstoffperoxid und insbesondere Lösungen enthaltend Natriumcarbonat, Natriumbicarbonat oder Natriumhydroxid.

Geeignete Verfahren sind beispielsweise genannt in M. Thiemann et. al in Ullmann's Encyclopedia, 6th Edition, 2000, Electronic Edition, Kapitel "Nitric Acid, Nitrous Acid, and Nitrogen Oxides", Abschnitt 1.4.2.3.

Allgemein erfolgt die NOₓ-Absorption in Einrichtungen in denen eine Gas-Flüssig-Phasengrenzfläche vorliegt, über die ein Stoff- und Wärmeübergang zwischen den Phasen ermöglicht wird, und die bei Bedarf mit internen oder externen Einrichtungen zur Wärmezufuhr und/oder Wärmeabfuhr versehen sind. Die Führung der Phasen im Absorber kann im Gleichstrom, im Gegenstrom oder einer Kombination der genannten erfolgen.

Die Absorption kann erfindungsgemäß ein- oder mehrstufig durchgeführt werden.

Die Absorption erfolgt erfindungsgemäß bei Temperaturen zwischen -20 und 100 °C, bevorzugt zwischen 0 und 60 °C, besonders bevorzugt zwischen 0 und 40 °C und bei Drücken zwischen 0,1 und 100 bar, bevorzugt zwischen 1 und 30 bar.

Mögliche Ausführungsformen des Absorbers sind Kolonnen mit Böden, beispielsweise Glockenböden oder Siebböden, Kolonnen mit strukturierten Einbauten wie beispielsweise Packungen, Kolonnen mit unstrukturierten Einbauten wie beispielsweise Füllkörpern, oder Apparate in denen die Flüssigphase dispergiert vorliegt, beispielsweise durch Versprühen in Düsen, oder eine Kombination der genannten.

In einer weiteren Ausführungsform betrifft die vorliegende Erfindung daher ein Verfahren wie oben beschrieben, wobei Schritt B die Absorption von Stickoxiden in saurer oder alkalischer Lösung umfasst.

Im Sinne der vorliegenden Erfindung erfolgt die Abtrennung von NOₓ bevorzugt durch Absorption in einer sauren oder einer alkalischen Lösung. Die Absorption wird zwischen -20 und 120°C, insbesondere zwischen -10 und 75°C, bevorzugt zwischen 0 und 60°C, beispielsweise zwischen 0 und 40 °C und bei einem Druck zwischen 0,2 und 100 bar, insbesondere zwischen 0,5 bis 50 bar, bevorzugt zwischen 1 und 10 bar durchgeführt.

Sofern die NOₓ-Konzentration im N₂O-haltigen Gasgemisch mehr als 1 Vol.-% beträgt, wird als Lösungsmittel für Schritt B bevorzugt wässrige Salpetersäure eingesetzt, mit einem HNO₃-Gehalt zwischen 0 und 69 Gew.-%, bevorzugt zwischen 0 und 10 Gew.-%. Vorteilhaft hierbei ist, dass die NOₓ-Abreicherung in der Gasphase mit der Herstellung von Salpetersäure mit 1-69 Gew.-% HNO₃ einhergeht. Im Sinne der weiteren Nutzbarkeit wird dabei bevorzugt eine Salpetersäure mit 30-60 Gew.-% HNO₃ hergestellt.

Diese Vorgehensweise kommt im Rahmen der vorliegenden Erfindung beispielsweise dann bevorzugt zum Einsatz, wenn das N₂O-haltige Eduktgas aus einem Carbonsäureprozess (z.B. Adipinsäure) stammt, wobei NOₓ-Konzentrationen von 1 bis 50 Vol.-% vorliegen. Die NOₓ-Abtrennung gemäß Schritt B wird in diesem Fall bevorzugt vor der N₂O-Aufkonzentrierung gemäß Schritt A1 und A2 durchgeführt.

Gemäß einer Ausführungsform der vorliegenden Erfindung kann an die Schritte A1 und A2 ein weiterer Schritt B anschließen, vorzugsweise eine chemische Wäsche, besonders bevorzugt mit Natriumcarbonat-Lösung oder Natronlauge.

Im Rahmen der vorliegenden Erfindung kann z.B. die chemische Carbonat-Wäsche durch eine selektive katalytische Reduktion mit Ammoniak ersetzt werden, bei der sich N₂O inert verhält. Diese sogenannte SCR-DeNOx- oder DeNOx-Technologie wird beispielsweise in Ullmann's Encyclopedia of Chemical Technology, Kapitel "Air", Abschnitt 7.2.3.1. "Catalytic Reduction of Nitrogen Oxides in Flue Gases and Process Off-Gases", von J. Wolf et al., 6. Ausgabe (Online Edition), 2000. beschrieben. Gemäß dieser bevorzugten Ausführungsform der vorliegenden Erfindung können NOₓ Konzentrationen von weniger als 100 ppm, vorzugsweise weniger als 50 ppm, beispielsweise weniger als 25 ppm und besonders bevorzugt von bis zu 5 ppm und sehr niedrige NH₃-Konzentrationen im Produkt erreicht werden, beispielsweise weniger als 10 ppm.

Gemäß einer besonders bevorzugten Ausführungsform der vorliegenden Erfindung kann das in das erfindungsgemäße Verfahren eingesetzte Gasgemisch enthaltend Distickstoffmonoxid aus einer Adipinsäureanlage stammen. Vorzugsweise wird das Abgas der AdipinsäureAnlage mit NO-Synthesegas gemischt und abgekühlt. Der Gasförmiger Strom wird dann komprimiert, vorzugsweise auf 7 bar, und gegebenenfalls mit Luft versetzt. Das heiße Gas nach der Kompression wird abgekühlt und in einen Absorptionsturm geleitet, in dem NOₓ abgereichert wird. Das Gas am Kopf der Kolonne hat vorzugsweise eine Temperatur von ca. 40°C bei einem Druck von 7 bar.

Das Abgas kann direkt in das erfindungsgemäße Verfahren eingesetzt werden. Gemäß einer weiter bevorzugten Ausführungsform kann jedoch das Abgas auf 100 bis 250°C, vorzugsweise 150 bis 200 °C, besonders bevorzugt auf 200°C aufgeheizt und zur Umsetzung gemäß Schritt B in die DeNOx-Anlage geführt werden.

Anschließend wird der Strom abgekühlt, komprimiert und wieder abgekühlt, bevor eine ein-oder mehrstufige Absorption/Desorption gemäß Schritt A1 und A2 durchgeführt wird.

Sofern eine NOₓ-Konzentration von < 1 Vol.-% im N₂O-haltigen Gasgemisch vorliegt, wie beispielsweise bei einem Abgas einer Salpetersäureanlage, wird als Absorbens für Schritt B bevorzugt eine alkalische Lösung eingesetzt. Im Sinne der vorliegenden Erfindung kommt diese Vorgehensweise bevorzugt zur Feinreinigung des N₂O-Gases nach der Aufkonzentrierung gemäß Schritt A1 und A2 zum Einsatz.

Neben den Schritten A1, A2 und B kann das erfindungsgemäße Verfahren auch weitere Schritte umfassen. So kann das Verfahren beispielsweise auch eine weitere Behandlung zwischen den Schritten A und dem Schritt B umfassen. Derartige Behandlungen umfassen beispielsweise eine Änderung der Temperatur oder eine Änderung des Drucks oder eine Änderung der Temperatur und des Drucks.

Erfindungsgemäß kann das durch das erfindungsgemäße Verfahren gereinigte Gasgemisch enthaltend Distickstoffmonoxid in einer weiteren Umsetzung eingesetzt werden. Dazu kann das Gasgemisch gasförmig eingesetzt werden. Es ist jedoch auch möglich, das erhaltene Gasgemisch zunächst derart zu behandeln, dass das Gasgemisch in flüssiger oder überkritischer Form vorliegt und dann in einer weiteren Umsetzung eingesetzt wird. Das Gasgemisch kann durch geeignete Wahl des Drucks oder der Temperatur verflüssigt werden.

Damit betrifft die vorliegende Erfindung auch ein Verfahren, wobei das erhaltene Gasgemisch verflüssigt wird.

Das nach einem erfindungsgemäßen Verfahren erhaltene Gasgemisch enthaltend Distickstoffmonoxid kann grundsätzlich für alle Anwendungen eingesetzt werden, bei denen üblicherweise reine Distickstoffmonoxidströme eingesetzt werden. Insbesondere eignen sich die Gasgemische beispielsweise für die Oxidation von Methanol zu Formaldehyd wie beispielsweise in der EP-A 0 624 565 oder DE-A 196 05 211 beschrieben. Daher betrifft die vorliegende Erfindung auch die Verwendung der nach einem erfindungsgemäßen Verfahren erhältlichen Gasgemische enthaltend Distickstoffmonoxid als Oxidationsmittel für Methanol.

Durch das erfindungsgemäße Verfahren werden Gasgemische enthaltend Distickstoffmonoxid erhalten, die einen besonders geringen Anteil an störenden und inerten Nebenkomponenten enthalten. Dies ist insbesondere für die Verwendung des Gasgemischs enthaltend Distickstoffmonoxid als Oxidationsmittel vorteilhaft, da durch den geringen Anteil an störenden oder inerten Nebenkomponenten kaum Nebenreaktionen auftreten und somit besonders reine Produkte erhalten werden können.

Grundsätzlich eignen sich die erfindungsgemäß erhältlichen Gasgemische enthaltend Distickstoffmonoxid zur Oxidation von Olefinen. Geeignete Olefine sind beispielsweise offenkettige oder cyclische Olefine mit einer oder mehreren Doppelbindungen. Weiter bevorzugt sind cyclische Olefine mit einer oder mehreren Doppelbindungen, beispielsweise Cyclopenten, Cyclohexen, Cyclohepten, Cycloocten, Cyclodecen, Cycloundecen, Cyclododecen, 1,4-Cyclohexadien, 1,5-Cyclooctadien, 1,5-Cyclododecadien oder 1,5,9-Cyclododecatrien.

Dieser angereicherte und gereinigte N₂O-haltige Gasstrom eignet sich ganz besonders zur Oxidation von Olefinen zu Ketonen. Für diesen Zweck kann vorzugsweise entweder das gasförmige N₂O direkt in das zu oxidierende Olefin oder ein anderes Lösungsmittel absorbiert werden oder das N₂O kann zuerst verflüssigt werden bevor es mit dem Olefin zur Reaktion gebracht wird.

Insbesondere beim Einsatz eines verflüssigten Gasgemischs enthaltend Distickstoffmonoxid ist es vorteilhaft, wenn der Anteil an Inertgasen im Gasgemisch möglichst gering ist, da ansonsten das Reaktorvolumen unnötig vergrößert wird.

Daher betrifft die vorliegende Erfindung auch ein Verfahren zur Herstellung eines Ketons, mindestens umfassend die folgenden Schritte
- A1: Absorprtion des Gasgemisches in einem organischen Lösungsmittel
- A2: Desorption des Gasgemisches aus dem beladenen organischen Lösungsmittel
- B: Einstellen des Gehalts an Stickoxiden NOₓ in dem Gasgemisch auf höchstens 0,5 Vol.-%, bezogen auf das Gesamtvolumen des Gasgemisches
- C: Inkontaktbringen des Gasgemisches mit mindestens einem Olefin, wobei des in Schritt C eingesetzte Gasgemisch verflüssigt ist.

Für die bevorzugten Ausführungsformen der Schritte A1, A2 und B gelten die vorstehenden Ausführungen. Auch für die Herstellung eines Ketons kann dabei die Reihenfolge der Schritte A1, A2 einerseits und B andererseits variieren. Erfindungsgemäß kann dabei der Schritt B nach den Schritten A1 und A2 ausgeführt werden. Es ist jedoch ebenso möglich, dass der Schritt B vor den Schritten A1 und A2 ausgerührt wird. In jedem Fall wird jedoch der Schritt C nach den Schritten A1, A2 und B ausgeführt.

Grundsätzlich ist es möglich, den Schritt B vor den Schritten A1 und A2 auszuführen, Ebenso ist es jedoch im Rahmen der vorliegenden Erfindung möglich, dass der Schritt B nach den Schritten A1 und A2 ausgeführt wird. Daher betrifft die vorliegende Erfindung in einer weiteren Ausführungsform auch ein Verfahren zur Herstellung eines Ketons wie oben beschrieben, bei dem die Schritte A1 und A2 vor dem Schritt B ausgeführt werden. Gemäß einer alternativen Ausführungsform betrifft die vorliegende Erfindung auch ein Verfahren zur Herstellung eines Ketons, bei dem der Schritt B vor den Schritte A1 und A2 ausgeführt wird

Ebenso ist es im Rahmen der vorliegenden Erfindung möglich, dass das Verfahren mehrere Schritte A1 und A2 oder mehrere Schritte B umfasst, wobei der Schritt B vor oder nach den Schritten A1 und A2 durchgeführt werden kann.

Die Umsetzung gemäß Schritt C kann generell gemäß sämtlicher Verfahrensführungen erfolgen, bei denen das Olefin und das Gasgemisch enthaltend Distickstoffmonoxid miteinander reagieren. Insbesondere sind sowohl kontinuierliche Verfahrensführungen und Fahrweisen der Umsetzung als auch Batch-Reaktion möglich. Die Reaktionsbedingungen für Schritt C werden erfindungsgemäß so gewählt, dass eine Reaktion des mindestens einen Olefins mit dem erfindungsgemäß gereinigten Gasgemisch stattfindet. Druck und Temperatur können entsprechend gewählt werden. '

Dabei kann die Reaktion in Gegenwart eines geeigneten Lösungsmittels durchgeführt werden. Es ist erfindungsgemäß aber ebenso möglich, die Umsetzung gemäß Schritt C ohne den Zusatz eines Lösungsmittels durchzuführen.

Erfindungsgemäß ist es jedoch auch möglich, dass das Verfahren zur Herstellung eines Ketons weitere Schritte umfasst. So kann das Gasgemisch enthaltend Distickstoffmonoxid beispielsweise vor Schritt C und nach den Schritten A1, A2 und B behandelt werden. Eine mögliche Behandlung ist beispielsweise eine Änderung von Druck und/oder Temperatur des Gasgemischs. Eine weitere mögliche Behandlung ist beispielsweise die Absorption in einem Lösungsmittel, so dass das absorbierte Gasgemisch in Schritt C eingesetzt werden kann. Dabei kann das Lösungsmittel jedes geeignete Lösungsmittel sein. Bevorzugt handelt es sich bei dem Lösungsmittel um das Olefin, das gemäß Schritt C oxidiert werden soll.

Im Rahmen der vorliegenden Erfindung wird das Gasgemisch enthaltend Distickstoffmonoxid vor Schritt C und nach den Schritten A1, A2 und B durch geeignete Wahl des Drucks und/oder der Temperatur verflüssigt. Das verflüssigte Gasgemisch enthaltend Distickstoffmonoxid wird dann gemäß Schritt C direkt mit dem Olefin in Kontakt gebracht.

Grundsätzlich können in Schritt C des erfindungsgemäßen Verfahrens alle geeigneten Olefine eingesetzt werden, beispielsweise Olefine mit 2 bis 18 C-Atomen, insbesondere Olefine mit 5 bis 12 C-Atomen. Geeignete Olefine sind beispielsweise offenkettige oder cyclische Olefine mit einer oder mehreren Doppelbindungen. Weiter bevorzugt sind cyclische Olefine mit einer oder mehreren Doppelbindungen, beispielsweise Cyclopenten, Cyclohexen, Cyclohepten, Cycloocten, Cyclodecen, Cycloundecen, Cyclododecen, 1,4-Cyclohexadien, 1,5-Cyclooctadien, 1,5-Cyclododecadien oder 1,5,9-Cyclododecatrien.

Besonders bevorzugt wird als Olefin Cyclopenten, Cyclododecen oder 1,5,9-Cyclododecatrien eingesetzt. Daher betrifft die vorliegende Erfindung in einer bevorzugten Ausführungsform ein Verfahren zur Herstellung eines Ketons wie oben beschrieben, wobei das Olefin ausgewählt ist aus der Gruppe bestehend aus Cyclopenten, Cyclododecen und 1,5,9-Cyclododecatrien.

Soweit die vorliegende Erfindung die Herstellung eines Ketons umfassend das Inkontaktbringen des erfindungsgemäß gereinigten Gasgemischs enthaltend Distickstoffmonoxid mit Cyclopenten betrifft, wird in Schritt C des erimdungsgemäßen Verfahrens ein Gemisch G(i) enthaltend Cyclopenten eingesetzt. Es wird ein Gemisch enthaltend Cyclopentanon erhalten. Im folgenden sind bevorzugte Verfahrensbedingungen dieser Ausführungsform beschrieben.

Grundsätzlich kann das Gemisch G(i) zusätzlich zu Cyclopenten jede weitere Verbindung enthalten. Geeignet sind unter anderem auch Verbindungen, die bei dem Inkontaktbringen gemäß Schritt C nicht mit N₂O reagieren können. Bevorzugt sind hierbei solche Verbindungen, die zwar prinzipiell mit N₂O reagieren können, bei den gemäß Schritt C gewählten Reaktionsbedingungen jedoch gegenüber N₂O inert sind. Der Begriff "inert", wie er im Rahmen dieser Ausführungsform der vorliegenden Erfindung verwendet wird, bezeichnet Verbindungen, die bei den gemäß Schritt C gewählten Reaktionsbedingungen mit N₂O entweder nicht reagieren oder im Vergleich zur Reaktion von Cyclopenten mit N₂O derart eingeschränkt reagieren, dass ihr Umsetzungsprodukt mit N₂O im aus Schritt C resultierenden Gemisch zu höchstens 15 Gew.-%, bevorzugt zu höchstens 10 Gew.-% und besonders bevorzugt zu höchstens 5 Gew.-%, jeweils bezogen auf das Gesamtgewicht der aus Schritt C resultierenden Mischung, enthalten ist.

Demgemäß betrifft die vorliegende Erfindung auch ein Verfahren, wie oben beschrieben, das dadurch gekennzeichnet ist, dass das Gemisch G(i) zusätzlich zu Cyclopenten mindestens eine beim Inkontaktbringen gemäß Schritt C gegenüber N₂O inerte Verbindung enthält.

Als inerte Verbindungen sind Alkane wie beispielsweise Hexan, Octan, Decan, Dodecan, Cyclopentan oder Cyclododecan oder Alkylbenzole wie beispielsweise Benzol, Toluol, Xylole, Ethylbenzol oder Ether wie beispielsweise Methyl-tert-Butylether, Tetrahydrofuran, Diethylether oder Ester wie beispielsweise Methylacetat, Ethylacetat, Methylbenzoat oder Nitrile wie beispielsweise Acetonitril, Benzonitril oder Alkohole wie beispielsweise Butanol, 2-Ethylhexanol, Ethanol oder Phenole wie beispielsweise Phenol, Kresole oder Amine wie beispielsweise Anilin, Triethylamin, N,N-Dimethylanilin oder Gemische aus zwei oder mehr der genannten Verbindungen oder zwei oder mehr Verbindungen der genannten Klassen geeignet.

Ganz besonders bevorzugt sind solche Verbindungen, die bei den gemäß Schritt C gewählten Reaktionsbedingungen mit N₂O nicht reagieren.

Im Rahmen einer diesbezüglich bevorzugten Ausführungsform des erfindungsgemäßen Verfahrens wird als Eduktgemisch G(i) ein Gemisch eingesetzt, das aus Spaltung und Partialhydrierung von Dicyclopentadien in Anwesenheit eines Lösungsmittels gewonnen wird und Cyclopenten enthält, wobei das Lösungsmittel aus den oben genannten inerten Verbindungen ausgewählt wird. Bevorzugt wird hierbei zur Partialhydrierung ein 2:1-Gemisch aus Dicyclopentadien und Toluol eingesetzt. Dieses Verfahren ist beispielsweise in der JP 2000053597 A beschrieben. Gemäß der JP 200053597 wird Cyclopentadien durch Thermolyse von Dicyclopentadien in Anwesenheit eines aromatischen Kohlenwasserstoffs, bevorzugt Toluol, erhalten, wobei die Umsetzungsrate 98% beträgt. Das erhaltene Gas wird in ein Edelstahlreaktionsrohr geleitet, das mit Palladium/Aluminiumoxid-Katalysator gefüllt ist. Das Gas am Auslass des Reaktionsrohres wird mit einem Kühler kondensiert.

Gemäß einer weiteren bevorzugten Ausführungsform des erfindungsgemäßen Verfahrens besteht das Gemisch G(i) zu mindestens 99 Gew.-%, bezogen auf das Gesamtgewicht des Gemisches G(i), aus Kohlenwasserstoffen. Neben den Kohlenwasserstoffen kann demgemäß das Gemisch G(i) noch zu höchstens 1 Gew.-% mindestens eine weitere Verbindung enthalten, wobei unter anderem mindestens eine der oben genannten, von Kohlenwasserstoffen verschiedene, inerten bevorzugten Verbindungen zu höchstens 1 Gew.-% enthalten sein kann. Auch andere Verbindungen können zu höchstens 1 Gew.-% unter der Maßgabe enthalten sein, dass sie die Umsetzung von Cyclopenten gemäß Schritt C nicht stören.

Der Begriff "Kohlenwasserstoffgemisch", wie er im Rahmen der vorliegenden Erfindung verwendet wird, bezeichnet ein Gemisch aus Verbindungen, von denen jede ein nicht substituierter Kohlenwasserstoff ist und daher nur aus den Atomen C und H besteht. Die im Rahmen der vorliegenden Erfindung eingesetzten Kohlenwasserstoffgemische enthalten dabei zu höchstens 1 Gew.-%, bezogen auf das Gesamtgewicht des jeweiligen Gemisches G(i), weitere Verbindungen. Weiter bevorzugt enthält das Gemisch zu höchstens 0,5 Gew.-%, weiter bevorzugt zu höchstens 0,1 Gew.-%, weiter bevorzugt zu höchstens 0,01 Gew.-% und ganz besonders bevorzugt zu höchstens 0,001 Gew.-% weitere Verbindungen. Insbesondere bevorzugt sind Gemische G(i), die im Rahmen der Messgenauigkeit der jeweils eingesetzten Untersuchungsmethoden keine weiteren Verbindungen enthalten.

Gemäß einer bevorzugten Ausführungsform ist das Gemisch G(i) bei den gemäß Schritt C gewählten Reaktionsbedingungen flüssig oder überkritisch. Unter anderem bevorzugt sind hierbei Gemische G(i), die bei Umgebungstemperatur und Umgebungsdruck flüssig sind. Hierbei sind etwa Gemische zu nennen, von denen jede enthaltene Verbindung bei Umgebungstemperatur und Umgebungsdruck flüssig ist. Ebenso sind Gemische denkbar, die bei Umgebungstemperatur und Umgebungsdruck flüssig sind und dabei mindestens eine Verbindung enthalten, die beispielsweise bei Umgebungstemperatur und Umgebungsdruck fest oder gasförmig ist, bei Umgebungstemperatur und Umgebungsdruck in dem Gemisch G(i) jedoch gelöst vorliegt.

Im Rahmen einer ebenfalls bevorzugten Ausführungsform des erfindungsgemäßen Verfahrens wird ein Gemisch G(i) eingesetzt, das zu mindestens 99 Gew.-% aus C₅-Kohlenwasserstoffen und Kohlenwasserstoffen mit mehr als 5 Kohlenstoffatomen besteht. Neben Cyclopenten können demgemäß mindestens ein weiterer C₅-Kohlenwasserstoff oder mindestens ein Kohlenwasserstoff mit mehr als 5 Kohlenstoffatomen oder ein Gemisch aus mindestens einem weiteren C₅-Kohlenwasserstoff und mindestens einem Kohlenwasserstoff mit mehr als 5 Kohlenstoffatomen in G(i) enthalten sein.

Demgemäß beschreibt die vorliegende Erfindung auch ein Verfahren, wie oben beschrieben, das dadurch gekennzeichnet ist, dass das Gemisch G(i) mindestens 99 Gew.-% C₅-Kohlenwasserstoffe und Kohlenwasserstoffe mit mehr als 5 Kohlenstoffatomen enthält.

Als unter anderem besonders bevorzugte Kohlenwasserstoffe mit mehr als 5 Kohlenstoffatomen werden die entsprechenden, bereits oben im Rahmen der inerten Verbindungen genannten Kohlenwasserstoffe eingesetzt.

Wie bereits oben erwähnt, werden als Eduktgemische G(i) bevorzugt solche Gemische eingesetzt, wie sie in großtechnischen Verfahren anfallen. Im Rahmen der vorliegenden Erfindung sind hierbei Gemische bevorzugt, die zu mindestens 95 Gew.-%, weiter bevorzugt zu mindestens 97 Gew.-% und besonders bevorzugt zu mindestens 99 Gew.-% aus C₅-, C₆- und C₇-Kohlenwasserstoffen bestehen.

Demgemäß betrifft die vorliegende Erfindung auch ein Verfahren, wie oben beschrieben, das dadurch gekennzeichnet ist, dass das Gemisch G(i) zu mindestens 99 Gew.-% C₅- und C₆- oder C₅- und C₇- oder C₅- und C₆- und C₇-Kohlenwasserstoffe enthält.

Im Rahmen der vorliegenden Erfindung kann dabei das Gemisch G(i) neben Cyclopenten entweder mindestens einen weiteren C₅-Kohlenwasserstoff oder mindestens einen C₆-Kohlenwasserstoff oder mindestens einen C₇-Kohlenwasserstoff oder ein Gemisch aus mindestens einem weiteren C₅-Kohlenwasserstoff und mindestens einem C₆-Kohlenwasserstoff oder ein Gemisch aus mindestens einem weiteren C₅-Kohlenwasserstoff und mindestens einem C₇-Kohlenwasserstoff oder ein Gemisch aus mindestens einem weiteren C₅-Kohlenwasserstoff und mindestens einem C₆-Kohlenwasserstoff und mindestens einem C₇-Kohlenwasserstoff enthalten.

Im Rahmen einer bevorzugten Ausführungsform des erfindungsgemäßen Verfahrens wird als Eduktgemisch G(i) ein Kohlenwasserstoffgemisch eingesetzt, das aus einem Steamcracker oder einer Raffinerie gewonnen wird und Cyclopenten enthält. In diesem Zusammenhang sind beispielsweise C₅-Schnitte aus Steamcracker-Anlagen bevorzugt, die im Wesentlichen nur C₅- und C₆-Kohlenwasserstoffe enthalten. Kohlenwasserstoffe mit mehr als 6 Kohlenstoffatomen sind in den großtechnisch anfallenden C₅-Schnitten nicht enthalten, die neben Cyclopenten beispielsweise 2-Buten, Isopentan, 1-Penten, 2-Methylbuten-1, trans-2-Penten, n-Pentan, cis-2-Penten, 2-Methylbuten-2, Cyclopentan, 2,2-Dimethylbutan, 2-Methylpentan, 3-Methylpentan, n-Hexan und Benzol umfassen. Im Allgemeinen enthält ein C₅-Schnitt aus einer Steamcracker-Anlage Cyclopenten im Bereich von 5 bis 60 Gew.-% und bevorzugt im Bereich von 15 bis 50 Gew.-%.

Daher beschreibt die vorliegende Erfindung auch ein Verfahren, wie oben beschrieben, das dadurch gekennzeichnet ist, dass das Gemisch G(i) zu mindestens 99 Gew.-% ein Gemisch aus C₅- und C₆-Kohlenwasserstoffen enthält.

Erfindungsgemäß kann dieses Gemisch aus im wesentlichen C₅- und C₆-Kohlenwasserstoffen, das bevorzugt als C₅-Schnitt aus einer Steamcracker-Anlage gewonnen wird, als solches eingesetzt werden. Bevorzugt wird das Gemisch aus im wesentlichen C₅- und C₆-Kohlenwasserstoffen vor der erfindungsgemäßen Umsetzung gemäß Schritt C einer Reinigung unterzogen, bei der wiederum bevorzugt im Vergleich zu Cyclopenten leichter siedende Verbindungen abgetrennt werden. Während hierbei sämtliche denkbaren Methoden einsetzbar sind, ist die destillative Auftrennung des Gemisches bevorzugt.

Im Rahmen der vorliegenden Erfindung werden hierbei bevorzugt Gemische G(i) erhalten, die zu höchstens 10 Gew.-% C₅- und/oder C₆-Kohlenwasserstoffe enthalten, die leichter sieden als Cyclopenten. Sollte im zu reinigenden Gemisch G(i) gegebenenfalls zusätzlich mindestens ein C₄-Kohlenwasserstoff enthalten sein, so werden durch die bevorzugt eingesetzte Destillation bevorzugt Gemische G(i) erhalten, die zu höchstens 10 Gew.-% C₄- und/oder C₅- und/oder C₆-Kohlenwasserstoffe enthalten, die leichter sieden als Cyclopenten. Im Rahmen der vorliegenden Erfindung werden hierbei besonders bevorzugt Gemische G(i) erhalten, die zu höchstens 5 Gew.-%, weiter bevorzugt zu höchstens 3 Gew.-% und besonders bevorzugt zu höchstens 2 Gew.-% C₅- und/oder C₆-Kohlenwasserstoffe enthalten, die leichter sieden als Cyclopenten. Sollte im zu reinigenden Gemisch G(i) gegebenenfalls zusätzlich mindestens ein C₄-Kohlenwasserstoff enthalten sein, so werden durch die bevorzugt eingesetzte Destillation bevorzugt Gemische G(i) erhalten, die zu höchstens 5 Gew.-%, weiter bevorzugt zu höchstens 3 Gew.-% und besonders bevorzugt zu höchstens 2 Gew.-% C₄- und/oder C₅- und/oder C₆-Kohlenwasserstoffe enthalten, die leichter sieden als Cyclopenten.

Demgemäß beschreibt die vorliegende Erfindung auch ein Verfahren, wie oben beschrieben, das dadurch gekennzeichnet ist, dass das Gemisch G(i) mindestens 99 Gew.-%, bezogen auf das Gesamtgewicht des Gemisches G(i), an C₅- und C₆-Kohlenwasserstoffen und höchstens 2 Gew.-%, bezogen auf das Gesamtgewicht des Gemisches G(i), an im Vergleich zu Cylopenten leichter siedenden Kohlenwasserstoffen enthält.

Im Rahmen einer ebenfalls bevorzugten Ausführungsform des erfindungsgemäßen Verfahrens wird ein Gemisch G(i) eingesetzt, das zu mindestens 99 Gew.-% aus C₅- und C₇-Kohlenwasserstoffen besteht. Neben Cyclopenten können demgemäß mindestens ein weiterer C₅-Kohlenwasserstoff oder mindestens ein C₇-Kohlenwasserstoff oder ein Gemisch aus mindestens einem weiteren C₅-Kohlenwasserstoff und mindestens einem C₇-Kohlenwasserstoff in G(i) enthalten sein.

Demgemäß beschreibt die vorliegende Erfindung auch ein Verfahren, wie oben beschrieben, das dadurch gekennzeichnet ist, dass das Gemisch G(i) mindestens 99 Gew.-% C₅- und C₇-Kohlenwasserstoffe enthält

Als C₇-Kohlenwasserstoff sei beispielsweise besonders bevorzugt Toluol genannt.

Im Rahmen einer diesbezüglich bevorzugten Ausführungsform des erfindungsgemäßen Verfahrens wird als Eduktgemisch G(i) ein Kohlenwasserstoffgemisch eingesetzt, das aus Spaltung und Partialhydrierung von Dicyclopentadien in Anwesenheit von Toluol als Lösungsmittel gewonnen wird und Cyclopenten enthält. Bevorzugt wird hierbei zur Partialhydrierung ein 2:1-Gemisch aus Dicyclopentadien und Toluol eingesetzt. Dieses Verfahren ist beispielsweise in der JP 2000053597 A beschrieben.

Die derart erhaltenen Gemische enthalten im allgemeinen Cyclopenten in einem Bereich von 25 bis 75 Gew.-%, bevorzugt in einem Bereich von 35 bis 65 Gew.-% und besonders bevorzugt in einem Bereich von 40 bis 60 Gew.-%. Neben Cyclopenten enthalten die Reaktionsgemische hauptsächlich Cyclopentan und Toluol. Im allgemeinen besteht das aus Spaltung und Partialhydrierung eines Gemisches aus Dicyclopentadien und Toluol gewonnene Gemisch, das als Gemisch G(i) im Rahmen des erfindungsgemäßen Verfahrens eingesetzt werden kann, zu mindestens 99 Gew.-% aus Cyclopenten, Toluol und Cyclopentan.

Das gemäß dieser bevorzugten Ausführungsform erhaltene Gemisch, das zu mindestens 99 Gew.-% aus Cyclopenten, Toluol und Cyclopentan besteht, kann als solches eingesetzt werden.

Gemäß einer weiter bevorzugten Ausführungsform wird das aus der Spaltung und Partialhydrierung eines Gemisches aus Dicyclopentadien und Toluol gewonnene Gemisch vor dem Einsatz als Gemisch G(i) im erfindungsgemäßen Verfahren mindestens einer destillativen Trennung unterzogen, in dem ein Leichtsiedergemisch erhalten wird, das Cyclopenten im allgemeinen im Bereich von 60 bis 95 Gew.-%, bevorzugt im Bereich von 70 bis 90 Gew.-% und besonders bevorzugt im Bereich von 75 bis 85 Gew.-% enthält. Weiter enthält dieses Leichtsiedergemisch Toluol im allgemeinen im Bereich von höchstens 20 Gew.-%, bevorzugt von höchstens 10 Gew.-% und besonders bevorzugt von höchstens 5 Gew.-%, und Cyclopentan im allgemeinen im Bereich von 5 bis 25 Gew.-%, bevorzugt im Bereich von 7 bis 22 Gew.-% und besonders bevorzugt im Bereich von 10 bis 20 Gew.-%. Dieses Leichtsiedergemisch wird dann im erfindungsgemäßen Verfahren als Gemisch G(i) eingesetzt.

Weiter bevorzugt enthält das im Rahmen des erfindungsgemäßen Verfahrens eingesetzte Gemisch G(i) Cyclopenten in einem Bereich von 30 bis 90 Gew.-%, besonders bevorzugt in einem Bereich von 40 bis 90 Gew.-%, weiter besonders bevorzugt in einem Bereich von 45 bis 90 Gew.-% und insbesondere bevorzugt in einem Bereich von 50 bis 85 Gew.-%, jeweils bezogen auf das Gesamtgewicht des Gemisches G(i).

Insbesondere betrifft die vorliegende Erfindung daher auch die Verwendung eines cyclopentenhaltigen Kohlenwasserstoffgemisches als Edukt zur Herstellung von Cyclopentanon, **dadurch gekennzeichnet, dass** das cyclopentenhaltige Kohlenwasserstoffgemisch entweder der C₅-Schnitt einer Steamcracker-Anlage oder das aus der Partialhydrierung von Cyclopentadien erhaltene und Cyclopenten enthaltende Gemisch oder ein Gemisch des C₅-Schnittes einer Steamcracker-Anlage und dem aus der Partialhydrierung von Cyclopentadien erhaltenen und Cyclopenten enthaltenden Gemisch ist.

Die Umsetzung gemäß Schritt C kann generell gemäß sämtlicher Verfahrensführungen erfolgen, bei denen aus dem Gemisch G(i) enthaltend Cyclopenten und dem Gasgemisch enthaltend Distickstoffmonoxid Cyclopentanon entsteht. Insbesondere sind kontinuierliche Verfahrensführungen und Fahrweisen der Umsetzung als Batch-Reaktion möglich.

Gemäß einer bevorzugten Ausführungsform erfolgt die Umsetzung gemäß Schritt C in Batchfahrweise. Wiederum bevorzugt wird hierbei das Gemisch G(i) in einem geeigneten Reaktionsgefäß vorgelegt. Nachdem die Umsetzung, wie unten beschrieben, bevorzugt bei höheren Drücken als Atmosphärendruck erfolgt, wird als Reaktionsgefäß bevorzugt ein Autoklav verwendet.

Das Gemisch G(i) wird im Allgemeinen bei Temperaturen im Bereich von 0 bis 320 °C, bevorzugt im Bereich von 180 bis 300 °C und besonders bevorzugt im Bereich von 200 bis 290 °C vorgelegt. Die Drücke liegen im Allgemeinen im Bereich von 1 bis 500 bar, bevorzugt im Bereich von 10 bis 365 bar und besonders bevorzugt im Bereich von 25 bis 250 bar.

Nachdem das Gemisch G(i) bei den oben angegebene Temperaturen und Drücken vorgelegt wurde, wird es mit dem erfindungsgemäß gereinigten Gasgemisch enthaltend Distickstoffmonoxid in Kontakt gebracht, wobei die im Reaktionsgefäß vorhandene Luft vor dem Inkontaktbringen durch eine geeignete Maßnahme zumindest teilweise entfernt werden kann. Bevorzugt wird das Reaktionsgefäß mit mindestens einem Gas oder Gasgemisch gespült, wobei beispielsweise mit Stickstoff oder einem anderen geeigneten Inertgas oder einem Gemisch aus zwei oder mehr dieser Gase gespült werden kann. Besonders bevorzugt wird als Spülgas Stickstoff verwendet.

Das Gemisch G(i) und das erfindungsgemäß gereinigte Gasgemisch enthaltend N₂O werden in Mengen eingebracht, bei denen das molare Verhältnis von Cyclopenten zu N₂O im allgemeinen im Bereich von 0,05 bis 5, bevorzugt im Bereich von 0,5 bis 3 und besonders bevorzugt im Bereich von 0,9 bis 1,5 liegt.

Zum Inkontaktbringen der Gemische G(i) und des erfindungsgemäß gereinigten Gasgemischs enthaltend Distickstoffmonoxid wird letzteres im Allgemeinen mit einem Druck im Bereich von 5 bis 500 bar, bevorzugt im Bereich von 10 bis 365 bar und besonders bevorzugt im Bereich von 25 bis 250 bar in das Reaktionsgefäß eingebracht. Die Temperaturen, bei denen das Inkontaktbringen erfolgt, werden hierbei durch geeignete Maßnahmen derart eingestellt, dass die Umsetzung des im Gemisch G(i) enthaltenen Cyclopenten mit dem im erfindungsgemäß gereinigten Gasgemisch enthaltenen N₂O bevorzugt in flüssiger oder überkritischer Phase stattfindet. Demgemäß liegen die Temperaturen, bei denen die Umsetzung stattfindet, im Allgemeinen im Bereich von 150 bis 320 °C, bevorzugt im Bereich von 180 bis 300 °C und besonders bevorzugt im Bereich von 200 bis 290 °C.

Gemäß einer besonders bevorzugten Ausführungsform des erfindungsgemäßen Verfahrens wird zunächst das erfindungsgemäß gereinigte Gasgemisch bei den oben angegebenen Drücken in das Reaktionsgefäß eingebracht und anschließend die Temperatur im Reaktionsgefäß mit einer Rate von im allgemeinen 1 bis 10 °C/min, bevorzugt von 1,5 bis 5 °C/min und besonders bevorzugt von 2 bis 4 °C/min erhöht.

Ist die Temperatur soweit erhöht, dass die oben angegebene, zur Umsetzung erforderlichen Temperatur erreicht ist, wird diese Temperatur im allgemeinen für einen Zeitraum im Bereich von 1 bis 48 h, bevorzugt im Bereich von 2 bis 30 h und besonders bevorzugt im Bereich von 5 bis 25 h aufrechterhalten. Dabei ist es denkbar, die Temperatur nicht konstant zu halten, sondern in den oben angegebenen Grenzen geeignet zu variieren.

Demgemäß betrifft die vorliegende Erfindung auch ein Verfahren, wie oben beschrieben, das dadurch gekennzeichnet ist, dass der Schritt C mindestens folgende Stufen (a) bis (d) umfasst:
(a) Einbringen des Gemisches G(i) in ein Reaktionsgefäß bei einer Temperatur im Bereich von 0 bis 320 °C und einem Druck im Bereich von 1 bis 500 bar;
(b) Inkontaktbringen des Gemisches G(i) in dem Reaktionsgefäß mit dem erfindungsgemäß gereinigten Gasgemisch enthaltend Distickstoffmonoxid bei einem Druck im Bereich von 5 bis 500 bar;
(c) Erhöhen der Temperatur des gemäß (b) erhaltenen Gemisches mit einer Rate im Bereich von 1 bis 10 °C/min auf eine Temperatur im Bereich von 150 bis 320 °C;
(d) Halten der gemäß (c) eingestellten Temperatur für einen Zeitraum im Bereich von 0,1 bis 48 h.

Nach erfolgter Umsetzung des Cyclopenten mit N₂O wird das sich im Reaktionsgefäß unter Druck befindliche Gemisch abgekühlt. Das Innere des Reaktionsgefäßes wird dabei gleichzeitig oder nach der Abkühlung oder sowohl während und nach der Abkühlung entspannt.

Neben der oben beschriebenen Umsetzung in einem Batchreaktor kann das erfmdungsgemäße Verfahren grundsätzlich in jedem anderen dafür geeigneten Reaktor durchgeführt werden. Ebenso ist die Kombination aus zwei oder mehr gleichen oder verschiedenen Reaktoren möglich. Unter anderem kann die Umsetzung gemäß Schritt C beispielsweise in mindestens einer Blasensäule durchgeführt werden. Bevorzugt wird die Umsetzung gemäß Schritt C in mindestens einem kontinuierlichen Reaktor durchgeführt. Beispielsweise kann die Umsetzung gemäß Schritt C in einem CSTR (continuous stirred tank reactor) oder in einer CSTR-Kaskade erfolgen. Weiter bevorzugt ist mindestens einer der kontinuierlichen Reaktoren ein kontinuierlicher Rohrreaktor.

Demgemäß betrifft die vorliegende Erfindung auch ein Verfahren, wie oben beschrieben, das dadurch gekennzeichnet ist, dass gemäß Schritt C die Gemische G(i) und das erfindungsgemäß gereinigte Gasgemisch enthaltend Distickstoffmonoxid in einem kontinuierlichen Rohrreaktor in Kontakt gebracht werden.

In einer weiter bevorzugten Ausführungsform ist mindestens einer der erfindungsgemäß eingesetzten kontinuierlichen Rohrreaktoren ein Rohrbündelreaktor.

Die Gemische G(i) und das erfindungsgemäß gereinigte Gasgemisch enthaltend Distickstoffmonoxid können im Wesentlichen unter sämtlichen geeigneten Reaktionsbedingungen in den kontinuierlichen Reaktoren in Kontakt gebracht werden, die es erlauben, dass Cyclopenten mit N₂O zu Cyclopentanon reagiert. Insbesondere bevorzugt werden die Reaktionsbedingungen in dem mindestens einen kontinuierlichen Reaktor so gewählt, dass die Umsetzung gemäß Schritt C in flüssiger oder überkritischer Phase erfolgt. Weiter bevorzugt sind Reaktionsbedingungen, bei denen der gesamte Reaktorinhalt flüssig ist. Unter dem Begriff "Reaktorinhalt" werden dabei die Gemische G(i) und das erfindungsgemäß gereinigte Gasgemisch verstanden, nachdem sie in den Reaktor eingebracht wurden, sowie die aus diesen Gemischen resultierende Mischung.

Insbesondere bevorzugt werden die Gemische G(i) und das erfindungsgemäß gereinigte Gasgemisch getrennt voneinander in den Reaktor eingeführt.

Ebenso ist es im Rahmen der vorliegenden Erfindung jedoch möglich, das erfindungsgemäß gereinigte Gasgemisch enthaltend Distickstoffmonoxid und das Gemisch G(i) oder einen Teil des Gemischs G(i) zu mischen und dieses Gemisch in den Reaktor einzuführen. Erfindungsgemäß werden das erfindungs gemäß gereinigte Gasgemisch enthaltend Distickstoffmonoxid und das Gemisch G(i) oder einen Teil des Gemischs G(i) bei Temperaturen gemischt, bei denen keine Reaktion stattfindet. Vorzugsweise erfolgt das Mischen bei einer Temperatur im Bereich von 80 bis 200°C, bevorzugt im Bereich von 90 bis 150°C, insbesondere im Bereich von 100 bis 120 °C.

Demgemäß betrifft die vorliegende Erfindung auch ein Verfahren, wie oben beschrieben, das dadurch gekennzeichnet ist, dass der kontinuierliche Rohrreaktor während der Umsetzung gemäß Schritt C im wesentlichen ausschließlich mit Flüssigkeit gefüllt ist.

Ganz besonders bevorzugt werden die Reaktionsbedingungen so gewählt, dass das sich im Reaktor befindliche Gemisch homogen und einphasig ist.

Das Gemisch G(i) wird im allgemeinen bei Temperaturen im Bereich von 0 bis 320 °C, bevorzugt im Bereich von 180 bis 300 °C und besonders bevorzugt im Bereich von 200 bis 290 °C in den kontinuierlichen Reaktor eingebracht, wobei die Drücke im allgemeinen im Bereich von 1 bis 500 bar, bevorzugt im Bereich von 10 bis 365 bar und besonders bevorzugt im Bereich von 25 bis 300 bar liegen.

Das erfindungsgemäß gereinigte Gasgemisch enthaltend Distickstoffmonoxid wird im allgemeinen bei Temperaturen im Bereich von 0 bis 320 °C, bevorzugt im Bereich von 180 bis 300 °C und besonders bevorzugt im Bereich von 200 bis 290 °C in den kontinuierlichen Reaktor eingebracht, wobei die Drücke im allgemeinen im Bereich von 5 bis 500 bar, bevorzugt im Bereich von 10 bis 365 bar und besonders bevorzugt im Bereich von 25 bis 300 bar liegen.

Im kontinuierlichen Reaktor werden die Gemische G(i) und das erfindungsgemäß gereinigte Gasgemisch enthaltend Distickstoffmonoxid in Kontakt gebracht. Das Gemisch G(i) und das erfindungsgemäß gereinigte Gasgemisch werden in Mengen eingebracht, bei denen das molare Verhältnis von Cyclopenten zu N₂O im allgemeinen im Bereich von 0,05 bis 10, bevorzugt im Bereich von 0,5 bis 7 und besonders bevorzugt im Bereich von 1 bis 5 liegt.

Die Umsetzung des im Gemisch G(i) enthaltenen Cyclopenten mit dem im erfindungsgemäß gereinigten Gasgemisch enthaltenen N₂O in dem mindestens einen kontinuierlichen Reaktor erfolgt bei Temperaturen im allgemeinen im Bereich von 150 bis 320 °C, bevorzugt im Bereich von 180 bis 300 °C und besonders bevorzugt im Bereich von 200 bis 290 °C. Die Drücke im kontinuierlichen Reaktor liegen dabei im allgemeinen im Bereich von 5 bis 500 bar, bevorzugt im Bereich von 10 bis 400 bar und besonders bevorzugt im Bereich von 100 bis 365 bar.

Die Verweilzeit des Reaktionsgemisches im kontinuierlichen Reaktor liegt im Allgemeinen im Bereich von 0,1 bis 48 h, bevorzugt im Bereich von 0,2 bis 5 h und besonders bevorzugt im Bereich von 0,3 bis 2,5 h. Dabei ist es denkbar, die Temperatur oder den Druck oder beide im Reaktor nicht konstant zu halten, sondern in den oben angegebenen Grenzen geeignet zu variieren.

Demgemäß beschreibt die vorliegende Erfindung auch ein Verfahren, wie oben beschrieben, das dadurch gekennzeichnet ist, dass der Schritt C mindestens folgende Stufen (aa) bis (dd) umfasst:
(aa) Einbringen des Gemisches G(i) in einen kontinuierlichen Reaktor bei einer Temperatur im Bereich von 0 bis 320 °C und einem Druck im Bereich von 1 bis 500 bar;
(bb) Einbringen des erfindungsgemäß gereinigten Gasgemischs enthaltend Distickstoffmonoxid in den kontinuierlichen Reaktor bei einer Temperatur im Bereich von 0 bis 320 °C und einem Druck im Bereich von 5 bis 500 bar;
(cc) Inkontaktbringen des Gemisches G(i) mit dem erfindungsgemäß gereinigten Gasgemisch in dem kontinuierlichen Reaktor bei einer Temperatur im Bereich von 100 bis 320 °C und einem Druck im Bereich von 5 bis 500 bar; (dd) Umsetzung der Gemische G(i) und des erfindungsgemäß gereinigten Gasgemischs während einer Verweilzeit der Reaktionsmischung in dem kontinuierlichen Reaktor im Bereich von 0,1 bis 48 h.

Durch das erfindungsgemäße Verfahren gemäß der oben beschriebenen Verfahrensführungen unter Verwendung der Gemische G(i) und des erfindungsgemäß gereinigten Gasgemischs werden Cyclopentenumsätze erreicht, die im Allgemeinen bei mindestens 10 %, bevorzugt bei mindestens 20 % und weiter bevorzugt bei mindestens 50 % liegen. Die Obergrenze der Umsätze liegt im Allgemeinen bei 98 %, bevorzugt bei 99 % und besonders bevorzugt bei 99,9 %.

Die Cyclopentanonselektivitäten der Umsetzung bezüglich Cyclopenten liegen hierbei im Allgemeinen im Bereich von 92 bis 99,5 %.

Demgemäß beschreibt die vorliegende Erfindung auch ein Verfahren, wie oben beschrieben, das dadurch gekennzeichnet ist, dass das eingesetzte Cyclopenten im Bereich von 10 bis 99,9 %, bezogen auf die eingesetzte Gesamtmenge an Cyclopenten, mit einer Cyclopentanonselektivität bezüglich Cyclopenten im Bereich von 92 bis 99,5 % umgesetzt wird.

Das gemäß Schritt C erhaltene und Cyclopentanon enthaltende Gemisch kann im weiteren gemäß sämtlicher geeigneter Verfahren zur Gewinnung des Cyclopentanons aufgearbeitet werden. Besonders bevorzugt sind hierbei destillative Verfahren zu nennen.

Soweit die vorliegende Erfindung die Herstellung eines Ketons umfassend das Inkontaktbringen des erfindungsgemäß gereinigten Gasgemischs enthaltend Distickstoffmonoxid mit Cyclododecen betrifft, wird in Schritt C des erfindungsgemäßen Verfahrens Cyclododecen eingesetzt. Es wird ein Gemisch enthaltend Cyclododecanon erhalten. Im folgenden werden bevorzugte Verfahrensbedingungen dieser Ausführungsform beschrieben. Cyclododecen kann vorzugsweise durch Partialhydrierung aus Cyclododecatrien hergestellt werden. In dem erfindungsgemäßen Verfahren kann reines Cyclododecen oder ein Gemisch, enthaltend Cyclododecen, eingesetzt werden. Weiterhin kann Cyclododecen als cis-Isomer oder als trans-Isomer oder als Mischung aus cis- und trans-Isomer vorliegen.

Im Rahmen der erfindungsgemäßen Umsetzung des Cyclododecens mit dem erfindungsgemäß gereinigten Gasgemisch enthaltend Distickstoffmonoxid kann mindestens ein geeignetes Lösungsmittel oder Verdünnungsmittel eingesetzt werden. Als solche sind unter anderem Cyclododecan oder Cyclododecanon zu nennen, wobei im Wesentlichen alle gängigen Lösungs- und/oder Verdünnungsmittel geeignet sind unter der Maßgabe, dass sie weder eine C-C-Doppelbindung noch eine C-C-Dreifachbindung noch eine Aldehydgruppe aufweisen.

Im Allgemeinen ist bei der erfindungsgemäßen Umsetzung gemäß Schritt C der Zusatz eines Lösungsmittels oder Verdünnungsmittel nicht notwendig.

Die Umsetzung des Cyclododecens gemäß Schritt C kann kontinuierlich oder in Batch-Fahrweise durchgeführt werden, wobei auch Kombinationen aus kontinuierlicher und Batch-Fahrweise möglich sind. Bevorzugt ist die kontinuierliche Verfahrensführung.

Als Reaktoren sind sämtliche geeigneten Reaktoren zu nennen. Beispielsweise kann die Umsetzung von Cyclododecen gemäß Schritt C in mindestens einem CSTR (Continuous Stirred Tank Reactor) mit internem oder externem Wärmetauscher, in mindestens einem Rohrreaktor, in mindestens einem Schlaufenreaktor oder einer Kombination aus mindestens zwei dieser Reaktoren durchführt werden. Ebenso ist es möglich, mindestens einen dieser Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Denkbar ist auch ein axiales Temperaturprofil, das beispielsweise durch Gleichstromkühlung und entsprechende Einstellung der Kühlmittelmenge zu realisieren ist

Besonders bevorzugt wird die Oxidation des Cyclododecens in mindestens einem Rohrreaktor durchgeführt.

Demgemäß beschreibt die vorliegende Erfindung auch ein wie oben beschriebenes Verfahren, das dadurch gekennzeichnet ist, dass die Umsetzung des Cyclododecens mit Distickstoffmonoxid in mindestens einem Rohrreaktor durchgeführt wird.

Die Umsetzung des Cyclododecens erfolgt bevorzugt bei einer Temperatur im Bereich von 140 bis 350 °C, weiter bevorzugt im Bereich von 200 bis 325 °C und besonders bevorzugt im Bereich von 225 bis 300 °C.

Demgemäß betrifft die vorliegende Erfindung auch ein wie oben beschriebenes Verfahrens, das dadurch gekennzeichnet ist, dass die Umsetzung gemäß Schritt C kontinuierlich in mindestens einem Rohrreaktor bei einer Temperatur im Bereich von 140 bis 350 °C durchgeführt wird.

Der Druck im Reaktionsgefäß, bevorzugt in mindestens einem Rohrreaktor, liegt im Allgemeinen bei Werten, die größer oder gleich, bevorzugt größer dem Eigendruck des Eduktgemisches beziehungsweise des Produktgemisches bei der gewählten Reaktionstemperatur oder den gewählten Reaktionstemperaturen im Reaktionsgefäß sind. Im Allgemeinen liegen die Reaktionsdrücke im Bereich von 1 bis 14.000 bar, bevorzugt im Bereich vom Eigendruck bis 3000 bar, besonders bevorzugt im Bereich vom Eigendruck bis 1000 bar und insbesondere bevorzugt im Bereich vom Eigendruck bis 325 bar.

Die Verweilzeit des Reaktionsgutes im Reaktor liegt im Allgemeinen bei bis zu 30 h, bevorzugt im Bereich von 0,1 bis 30 h, weiter bevorzugt im Bereich von 0,25 bis 25 h und insbesondere bevorzugt im Bereich von 0,3 bis 20 h.

Das Molverhältnis der Edukte Distickstoffmonoxid : Cyclododecen liegt im Allgemeinen bei bis zu 0,05 bis 5, bevorzugt im Bereich von 0,07 bis 2, weiter bevorzugt im Bereich von 0,1 bis 2 und besonders bevorzugt im Bereich von 0,1 bis 1.

Insbesondere bevorzugt werden die Reaktionsbedingungen so gewählt, dass der Umsatz an Cyclododecen im Bereich von 5 bis 95 %, besonders bevorzugt im Bereich von 7 bis 80 % und insbesondere bevorzugt im Bereich von 10 bis 50 % liegt.

Der Begriff "Umsatz", wie er obenstehend verwendet wird, bezeichnet den Gesamtumsatz an Cyclododecen. Wird als Edukt ausschließlich cis-Cyclododecen oder ausschließlich trans-Cyclododecen eingesetzt, so entspricht der Gesamtumsatz dem Umsatz an dem jeweiligen Isomer. Wird als Edukt ein Gemisch aus cis- und trans-Isomer, enthaltend x Mol.-% cis-Isomer und y Mol.-% trans-Isomer, eingesetzt und das cis-Isomer zu m % und das trans-Isomer zu n % umgesetzt, so berechnet sich der Gesamtumsatz als Summe mx + ny.

Im Falle, dass als Edukt ein Isomerengemisch eingesetzt wird, ist es im Rahmen der vorliegenden Erfindung bevorzugt, die Umsetzung gemäß Schritt C in mindestens zwei Schritten, weiter bevorzugt in zwei oder drei Schritten und ganz besonders bevorzugt in zwei Schritten durchzuführen.

In einem ersten Schritt wird dabei eine Temperatur gewählt, die bevorzugt im Bereich von 140 bis 300 °C, weiter bevorzugt im Bereich von 180 bis 290 °C und insbesondere bevorzugt im Bereich von 225 bis 275 °C liegt. In diesem ersten Schritt wird hauptsächlich das trans-Isomer zu Cyclododecanon oxidiert. In einem zweiten Schritt wird eine im Vergleich zum ersten Schritt erhöhte Temperatur gewählt, die bevorzugt im Bereich von 165 bis 350 °C, weiter bevorzugt im Bereich von 225 bis 325 °C und insbesondere bevorzugt im Bereich von 275 bis 310 °C liegt. In diesem zweiten Schritt wird das cis-Isomer zu Cyclododecanon oxidiert.

Demgemäß betrifft die vorliegende Erfindung auch ein wie oben beschriebenes Verfahren, das dadurch gekennzeichnet ist, dass ein Gemisch, enthaltend cis-Cyclododecen und trans-Cyclododecen, mit Distickstoffmonoxid in zwei Stufen umgesetzt wird.

Ebenso betrifft die vorliegende Erfindung auch ein wie oben beschriebenes Verfahren, das dadurch gekennzeichnet ist, dass in der ersten Stufe die Umsetzung bei einer Temperatur im Bereich von 140 bis 300 °C und in der zweiten Stufe die Umsetzung bei einer Temperatur im Bereich von 165 bis 350 °C durchgeführt wird, wobei die Temperatur in der ersten Stufe niedriger ist als die Temperatur in der zweiten Stufe.

Was die weiteren Reaktionsparameter wie beispielsweise Druck, Verweilzeit oder Reaktionsgefäße der beiden Stufen des oben genannten bevorzugten zweistufigen Verfahrens anbelangt, so wird diesbezüglich auf die allgemeinen und bevorzugten Ausführungsformen des oben beschriebenen einstufigen Verfahrens verwiesen.

Das oben beschriebene zweistufige Verfahren kann gemäß sämtlicher geeigneter Verfahrensführungen realisiert werden. Beispielsweise kann das zweistufige Verfahren in mindestens zwei Reaktoren durchgeführt werden, wobei in mindestens einem Reaktor die niedrigere Temperatur und in mindestens einem weiteren Reaktor die höhere Temperatur eingestellt wird. Ebenso ist es möglich, die unterschiedlichen Temperaturen in einem einzigen Reaktor, der mindestens zwei Zonen verschiedener Temperatur aufweist, zu realisieren. Wird ein Reaktor mit mindestens zwei Zonen verschiedener Temperatur eingesetzt, so können die beiden Temperaturen kontinuierlich oder diskontinuierlich ineinander übergehen. Beispielsweise besonderes bevorzugt ist hierbei ein Rohrreaktor mit einem axialen Temperaturprofil, das beispielsweise wie oben beschrieben zu realisieren ist.

Werden im Rahmen des zweistufigen Verfahrens mindestens zwei verschiedene Reaktoren eingesetzt, so kann zwischen mindestens zwei dieser Reaktoren mindestens eine Zwischenbehandlung des Reaktionsgutes erfolgen. Als mögliche Zwischenbehandlungen sind etwa zu nennen:
- Erwärmung des Reaktionsgutes;
- Änderung des Druckes, unter dem sich das Reaktionsgut befindet. Bevorzugt ist in diesem Zusammenhang beispielsweise die Erhöhung des Druckes über beispielsweise mindestens eine Pumpe und/oder mindestens einen Kompressor;
- Zudosierung mindestens eines Edukts. Insbesondere kann Distickstoffmonoxid und/oder Cyclododecen zudosiert werden. Im Falle des Cyclododecens kann es sich dabei um frisches Edukt und/oder um Cyclododecen handeln, das in der zweiten Stufe nicht umgesetzt wird und durch mindestens eine geeignete Maßnahme aus dem Produktstrom abgetrennt und in das Verfahren rückgeführt wird.
- Abtrennung von gebildetem Cyclododecanon durch mindestens eine geeignete Maßnahme wie beispielsweise bevorzugt durch mindestens einen destillativen Schritt.

Gemäß einer weiter bevorzugten Ausführungsform des erfindungsgemäßen Verfahrens wird im Falle, dass als Edukt ein Gemisch aus cis- und trans-Cyclododecen eingesetzt wird, mindestens ein Katalysator zugegeben, der in der Lage ist, unter den Reaktionsbedingungen, die für die Umsetzung des Cyclododecens gewählt werden, die Einstellung des Gleichgewichts zwischen cis- und trans-Isomer zu katalysieren.

Grundsätzlich können hierfür sämtliche geeigneten Katalysatoren eingesetzt werden. Besonders bevorzugt wird im erfindungsgemäßen Verfahren zu diesem Zweck mindestens ein Katalysator eingesetzt, wie er auch für Hydrierungen beispielsweise von Olefinen oder Polyenen verwendet wird. Insbesondere bevorzugt werden im erfindungsgemäßen Verfahren solche Isomerisierungskatalysatoren eingesetzt, die mindestens ein Übergangsmetall wie unter anderem bevorzugt Ru enthalten.

Die zur Gleichgewichtseinstellung zwischen cis- und trans-Isomer eingesetzten Isomerisierungskatalysatoren können entweder homogene oder heterogene Katalysatoren sein. Ebenso ist möglich, mindestens einen homogenen und mindestens einen heterogenen Katalysator einzusetzen. Die heterogenen Katalysatoren können hierbei als Suspensions- oder als Festbettkatalysator eingesetzt werden. Ebenso ist es möglich, sowohl mindestens einen heterogenen Suspensionskatalysator als auch mindestens einen heterogenen Festbettkatalysator, gegebenenfalls zusätzlich zu mindestens einem homogenen Katalysator, einzusetzen. Besonders bevorzugt wird mindestens ein homogener Katalysator eingesetzt.

Während grundsätzlich sämtliche geeigneten homogenen Katalysatoren eingesetzt werden können, werden bevorzugt solche verwendet, die Ru als aktives Metall enthalten. Weiter besonders bevorzugt werden Katalysatoren eingesetzt, wie sie in der US 5,180,870, US 5,321,176, US 5,177,278, US 3,804,914, US 5,210,349 US 5,128,296, US B 316,917 und in D.R. Fahey in J. Org. Chem. 38 (1973) S. 80-87 beschrieben sind. Solche Katalysatoren sind etwa (TPP)₂(CO)₃Ru, [Ru(CO)₄]₃, (TPP)₂Ru(CO)₂Cl₂, (TPP)₃(CO)RuH₂, (TPP)₂(CO)₂RuH₂, (TPP)₂(CO)₂RuClH, oder (TPP)₃(CO)RuCl₂.

Als ganz besonders bevorzugter Katalysator wird (TPP)₂(CO)₂RuCl₂ oder eine entsprechende Cl-freie Variante wie beispielsweise (TPP)₂(CO)₂RuH₂ eingesetzt, wobei TPP für Triphenylphosphin steht.

Gemäß einer weiter bevorzugten Ausführungsform wird der eingesetzte Katalysator im erfindungsgemäßen Verfahren in situ hergestellt. Bei dieser Herstellung in situ geht man beispielsweise bevorzugt von den Verbindungen Rutheniumchlorid, Rutheniumacetat, Rutheniumacetylacetonat oder anderen Ru-Verbindungen aus.

Im Allgemeinen wird der Oxidation außer der mindestens einen Ru-Komponente zusätzlich mindestens eine der Verbindungen NR₃, PR₃, AsR₃ oder SbR₃ zugesetzt, wobei R für einen Alkyl-, Aralkyl-, Alkaryl- oder Arylrest mit bevorzugt 1 bis 20 C-Atomen steht. Insbesondere bevorzugt ist im Rahmen der vorliegenden Erfindung hierbei Triphenylphosphin.

Im Rahmen einer weiteren Ausführungsform wird die Oxidation in Gegenwart mindestens einer Carbonsäure durchgeführt, wie dies in der DE 198 56 862 A1 beschrieben ist.

Als Carbonsäure können beispielsweise aliphatische, cycloaliphatische, aromatische oder araliphatische Carbonsäuren verwendet werden. Bevorzugt werden solche eingesetzt, die unter den Reaktionsbedingungen im Reaktionssystem löslich sind. Beispiele sind etwa C₁-C₂₀ Monocarbonsäuren, C₂-C₆ Dicarbonsäuren, Cyclohexylcarbonsäure, Benzoesäure, Terephthalsäure, Phthalsäure oder Phenylessigsäure. Besonders bevorzugte Säuren sind aliphatische Mono- und Dicarbonsäuren, insbesondere Essigsäure, Propionsäure sowie C₁₂-C₂₀-Fettsäuren, Bernsteinsäure und Adipinsäure.

Bei der in situ-Herstellung des Katalysators wird besonders bevorzugt noch mindestens eine CO-Quelle zugesetzt. Diese kann CO selbst sein. Weitere mögliche CO-Quellen sind beispielsweise Formaldehyd, Methanol, Ethanol oder andere geeignete primäre Alkohole wie beispielsweise Benzylalkohol oder Diole oder Polyole mit mindestens einer primären Alkoholgruppe wie beispielsweise Ethylenglykol, Propylenglykol oder Glycerin.

Aus der erfindungsgemäßen Oxidation des Cyclododecens resultiert im Allgemeinen ein Produktgemisch. Bevorzugt enthält dieses Produktgemisch im Bereich von 5 bis 95 Gew.-%, besonders bevorzugt von 7 bis 80 Gew.-% und insbesondere bevorzugt von 10 bis 75 Gew.-% Cyclododecanon, jeweils bezogen auf das Gesamtgewicht des Produktgemisches nach Abkühlung auf 20 °C und Entspannung auf Normaldruck.

Als weitere Bestandteile enthält das Produktgemisch gegebenenfalls den vor der Oxidationsstufe verwendeten und nicht abgetrennten Katalysator, nicht umgesetztes Cyclododecen sowie gegebenenfalls mit dem Edukt in die Oxidation eingebrachte Verbindungen wie beispielsweise Cyclododecan und bei der Umsetzung mit Distickstoffmonoxid gegebenenfalls mit umgesetzte Verbindungen, wie dies untenstehend beschrieben ist.

Der zur Umsetzung verwendete Isomerisierungskatalysator kann im Folgenden entweder in das Verfahren rückgeführt werden, verworfen werden oder aufgearbeitet werden, beispielsweise, um das im Katalysator enthaltene mindestens eine Metall zurückzugewinnen. Wird der Katalysator in das Verfahren rückgeführt, so kann dieser entweder in die Verfahrensstufe der Umsetzung mit Distickstoffmonoxid zurückgeführt werden oder in einen beliebigen anderen Schritt, den das erfindungsgemäße Verfahren zusätzlich aufweisen kann. Gemäß einer untenstehend beschriebenen besonders bevorzugten Ausführungsform weist das erfindungsgemäße Verfahren als solchen zusätzlichen Schritt die Partialhydrierung mindestens eines Cyclododecatriens auf, wobei die genannte Partialhydrierung weiter bevorzugt in Anwesenheit des gleichen Katalysators erfolgen kann, der als Isomerisierungskatalysator zur Gleichgewichtseinstellung zwischen cis- und trans-Isomer des Cyclododecens eingesetzt wird. Auch dieser Partialhydrierung kann demgemäß der abgetrennte Katalysator zugeführt werden, wobei der Katalysator vor der Zuführung einem geeigneten Regenerationsschritt unterworfen werden kann.

Das als Edukt eingesetzte Cyclododecen, das entweder als cis-Isomer oder als trans-Isomer oder als Gemisch aus cis- und trans-Isomer eingesetzt werden kann, kann grundsätzlich aus jeder beliebigen Quelle stammen.

Ganz besonders bevorzugt wird im Rahmen der vorliegenden Erfindung Cyclododecen durch Partialhydrierung mindestens eines Cyclododecatriens, bevorzugt durch Partialhydrierung mindestens eines 1,5,9-Cyclododecatriens wie beispielsweise cis,trans,trans-1,5,9-Cyclododecatrien oder cis,cis,trans-1,5,9-Cyclododecatrien oder all-trans-1,5,9-Cyclododecatrien und insbesondere aus cis,trans,trans-1,5,9-Cyclododecatrien hergestellt.

Diese bevorzugten Verbindungen können beispielsweise durch Trimerisierung von reinem 1,3-Butadien hergestellt werden, wie dies beispielsweise in T. Schiffer, G. Oenbrink, "Cyclodecatriene, Cyclooctadiene, and 4-Vinylcyclohexene", Ullmann's Encyclopedia of Industrial Chemistry, 6th Edition (2000), Electronic Release, Wiley VCH beschrieben ist. Im Rahmen dieses Verfahrens entstehen beispielsweise bei Trimerisierung in Anwesenheit von Ziegler-Katalysatoren cis,trans,trans-1,5,9-Cyclododeeatrien, cis,cis,trans-1,5,9-Cyclododecatrien und all-trans-1,5,9-Cyclododecatrien, wie dies beispielsweise in H. Weber et al. "Zur Bildungsweise von cis,trans,trans-Cyclododecatrien-(1.5.9) mittels titanhaltiger Katalysatoren" in: Liebigs Ann. Chem. 681 (1965) S.10-20 beschrieben ist. Während sämtliche dieser Cyclododecatriene im Rahmen des erfindungsgemäßen Verfahrens partialhydriert werden können, ist, wie oben beschrieben, die Umsetzung von cis,trans,trans-1,5,9-Cyclododecatrien im Rahmen des vorliegenden erfindungsgemäßen Verfahrens besonders bevorzugt. Dieses cis,trans,trans-1,5,9-Cyclododecatrien wird besonders bevorzugt gemäß des in dem oben erwähnten Artikel von Weber et al. hergestellt.

Demgemäß beschreibt die vorliegende Erfindung auch ein wie oben beschriebenes Verfahren, das dadurch gekennzeichnet ist, dass das Cyclododecatrien durch Trimerisierung von 1,3-Butadien unter Verwendung eines Titan-Katalysators hergestellt wird.

Während grundsätzlich sämtliche geeigneten Titan-Katalysatoren zur Trimerisierung eingesetzt werden können, ist der im Artikel von Weber et al. beschriebene Titantetrachlorid/Ethylaluminiumsesquichlorid-Katalysator besonders bevorzugt.

Das für die Trimerisierung eingesetzte Butadien weist insbesondere bevorzugt einen über Gaschromatographie bestimmten Reinheitsgrad von mindestens 99,6 % und weiter bevorzugt von mindestens 99,65 % auf. Insbesondere bevorzugt enthält das eingesetzte 1,3-Butadien im Rahmen der Nachweisgenauigkeit kein 1,2-Butadien und kein 2-Butin.

Aus dieser bevorzugten Trimerisierung werden im Allgemeinen Gemische erhalten, die mindestens 95 Gew.-%, bevorzugt mindestens 96 Gew.-% und weitere bevorzugt mindestens 97 Gew.-% cis,trans,trans-1,5,9-Cyclododecatrien enthalten. Beispielsweise insbesondere bevorzugt enthalten die Gemische in etwa 98 Gew.-% cis,trans,trans-1,5,9-Cyclododecatrien.

Daher betrifft die vorliegende Erfindung auch ein Verfahren zur Herstellung von Cyclododecanon, wobei das gemäß Schritt C eingesetzte Cyclododecen durch Partialhydrierung von Cyclododecatrien hergestellt wird.

Das dabei eingesetzte Cyclododecatrien wird vorzugsweise durch Trimerisierung von 1,3-Butadien hergestellt.

Die Trimerisierung erfolgt vorzugsweise in Anwesenheit eines Titan-Katalysators, das erhaltene Cyclododecatrien ist vorzugsweise cis,trans,trans-1,5,9-Cyclododecatrien.

Weiter beschreibt die vorliegende Erfindung auch ein integriertes Verfahren zur Herstellung von Cyclododecanon, das mindestens die folgenden Schritte (a) und (b) sowie (i) und (ii) umfasst:
(a) Herstellung von Cyclododecatrien aus 1,3-Butadien;
(b) Partialhydrierung des Cyclododecatriens unter Erhalt von Cyclododecen;
   (i) Bereitstellung eines Distickstoffmonoxid enthaltenden Gasgemisches, enthaltend jeweils von 0 bis 0,5 Vol.-% Sauerstoff und/oder Stickoxide, auf der Basis mindestens eines Abgasstroms einer Adipinsäureanlage und/oder einer Salpetersäureanlage und/oder einer Hydroxylaminanlage und/oder einer mit dem Abgas einer Adipinsäureanlage und/oder einer Dodecandisäureanlage und/oder einer Hydroxylaminanlage betriebenen Salpetersäureanlage;
   (ii) Umsetzung von gemäß (b) erhaltenem Cyclododecen mit dem gemäß (i) bereitgestellten Gasgemisch unter Erhalt von Cyclododecanon.

Verfahren zur katalytischen Partialhydrierung von Cyclododecatrien sind in der Literatur beschrieben. Dabei ist es im Allgemeinen essentiell, dass die Ausbeute bei dieser Reaktion sehr hoch ist, da sich aufgrund der geringen Massen- und Polaritätsunterschiede der Edukte und Produkte diese nicht oder nur sehr aufwändig destillativ voneinander trennen lassen. Der Cyclododecatrien-Umsatz muss daher möglichst quantitativ sein.

Die Hydrierung von Polyenen zu Monoenen an homogenen Ru-Katalysatoren unter Zusatz von Wasser ist beispielsweise in der US 5,180,870 beschrieben. In Beispiel 2 dieser Schrift wird unter Zusatz von Wasser nach 4 h Reaktionszeit ein Cyclododecatrien-Umsatz von 98,4 % erreicht. Welche Cyclododecen-Ausbeute erhalten wird, ist nicht beschrieben. In Beispiel 1 dieser Schrift wird unter Zusatz von etwas weniger Wasser als im Beispiel 2 nach 8 h Reaktionszeit ein nur unbefriedigender Umsatz von 85,8 % erreicht.

In der US 5,321,176 ist der Zusatz von Aminen zur homogen katalysierten Hydrierung beschrieben.

In der US 5,177,278 wird die Cyclododecatrien-Hydrierung mit homogenen Ru-Katalysatoren in Gegenwart von Lösungsmitteln wie Ethern oder Estern vorgenommen. Nach den Beispielen dieser Schrift liegen die besten Cyclododecen-Selektivitäten bei 96 - 98 %. Allerdings wird in keinem Fall quantitativer Umsatz erreicht, so dass sich bei der Aufarbeitung ein Trennproblem stellt.

In der US 3,925,494 wird ebenfalls in Lösungsmitteln gearbeitet. Die maximale Cyclododecen-Ausbeute ist mit ca. 95 % beschrieben. Allerdings ist auch hier der Umsatz nicht quantitativ.

Von D. R. Fahey wird in J. Org. Chem. 38 (1973) S. 80-87 die Hydrierung von Cyclododecatrien an verschiedenen homogenen Ru-Katalysatoren beschrieben. In allen Beispielen wird in Gegenwart größerer Mengen Lösungsmittel gearbeitet. Es werden Cyclododecen-Ausbeuten von ca. 98 % beschrieben. Allerdings ist die beschriebene Einsatzmenge an Ru, bezogen auf Cyclododecatrien, sehr hoch.

In der DE 198 56 862 A1 ist die Hydrierung von Cyclododecatrien an homogenen Ru-Katalysatoren in Gegenwart von Carbonsäuren beschrieben. Dabei können Cyclododecen-Ausbeuten von 98 % erreicht werden.

Im Rahmen der vorliegenden Erfindung kann die katalytische Partialhydrierung von Cyclododecatrien zu Cyclododecen gemäß sämtlicher geeigneter Methoden erfolgen.

Insbesondere kann die katalytische Partialhydrierung mit homogenen oder heterogenen Katalysatoren durchgeführt werden, wobei die heterogenen Katalysatoren als Suspension oder als Festbett eingesetzt werden können.

Als heterogene Katalysatorsysteme werden bevorzugt solche eingesetzt, die mindestens eines der Elemente Pd, Pt, Ru, Ir, Ni und Rh als aktives Hydriermetall enthalten.

Gemäß einer besonders bevorzugten Ausführungsform wird im erfindungsgemäßen Verfahren Cyclododecatrien in Anwesenheit mindestens eines homogenen Hydrierkatalysators zu Cyclododecen partiell hydriert.

Während grundsätzlich sämtliche geeigneten homogenen Katalysatoren eingesetzt werden können, werden bevorzugt solche verwendet, die Ru als aktives Hydriermetall enthalten. Weiter besonders bevorzugt werden Katalysatoren eingesetzt, wie sie in der US 5,180,870, US 5,321,176, US 5,177,278, US 3,804,914, US 5,210,349 US 5,128,296, US B 316,917 und in D.R. Fahey in J. Org. Chem. 38 (1973) S. 80-87 beschrieben sind. Solche Katalysatoren sind etwa (TPP)₂(CO)₃Ru, [Ru(CO)₄]₃, (TPP)₂Ru(CO)₂Cl₂, (TPP)₃(CO)RuH₂, (TPP)₂(CO)2RuH₂, (TPP)₂(CO)₂RuClH oder (TPP)₃(CO)RuCl₂.

Als ganz besonders bevorzugter Katalysator wird (TPP)₂(CO)₂RuCl₂ oder eine entsprechende Cl-freie Variante wie beispielsweise (TPP)₂(CO)₂RuH₂ eingesetzt, wobei TPP für Triphenylphosphin steht.

Gemäß einer weiter bevorzugten Ausführungsform wird der zur Partialhydrierung eingesetzte Katalysator im erfindungsgemäßen Verfahren in situ hergestellt. Bei dieser Herstellung in situ geht man beispielsweise bevorzugt von den Verbindungen Rutheniumchlorid, Rutheniumacetat, Rutheniumacetylacetonat oder anderen Ru-Verbindungen aus.

Im Allgemeinen wird der Hydrier-Reaktion außer der mindestens einen Ru-Komponente zusätzlich mindestens eine der Verbindungen NR₃, PR₃, AsR₃ oder SbR₃ zugesetzt, wobei R für einen Alkyl-, Aralkyl-, Alkaryl- oder Arylrest mit bevorzugt 1 bis 20 C-Atomen steht. Insbesondere bevorzugt ist im Rahmen der vorliegenden Erfindung hierbei Triphenylphosphin.

Bezogen auf 1 kg Cyclododecatrien werden im erfindungsgemäßen Verfahren, gerechnet als Metall, im allgemeinen 0,1 bis 2000 mg aktives Hydriermetall, besonders bevorzugt Ru, eingesetzt. Bevorzugt werden 1 bis 1000 mg und besonders bevorzugt 10 bis 200 mg eingesetzt.

Gemäß einer Ausführungsform des erfindungsgemäßen Verfahrens wird der Katalysator nach erfolgter Partialhydrierung aus dem Reaktionsgut abgetrennt. Der abgetrennte Katalysator wird gemäß einer weiteren Ausführungsform der vorliegenden Erfindung einem beliebigen Verfahren zugeführt und ganz besonders bevorzugt in das erfindungsgemäße Verfahren rückgeführt. Die Abtrennung des Katalysators erfolgt erfindungsgemäß besonders bevorzugt in mindestens einer Destillation, wobei das Produkt der Partialhydrierung, Cyclododecen, über Kopf und der Katalysator, gegebenenfalls mit Anteilen an Cyclododecen, über Sumpf abgetrennt wird.

Aufgrund der wie obenstehend beschrieben sehr niedrigen Mengen an Katalysatormaterial und der damit sehr niedrigen Kosten für den Katalysator ist es im erfindungsgemäßen Verfahren im Allgemeinen nicht notwendig, den Katalysator nach der Partialhydrierung aus der Reaktionsmischung abzutrennen und in das Verfahren rückzuführen.

Im Rahmen einer weiteren Ausführungsform wird die Partialhydrierung in Gegenwart mindestens einer Carbonsäure durchgeführt, wie dies in der DE 198 56 862 A1 beschrieben ist.

Als Carbonsäure können beispielsweise aliphatische, cycloaliphatische, aromatische oder araliphatische Carbonsäuren verwendet werden. Bevorzugt werden solche eingesetzt, die unter den Reaktionsbedingungen im Reaktionssystem löslich sind. Beispiele sind etwa C₁-C₂₀ Monocarbonsäuren, C₂-C₆ Dicarbonsäuren, Cyclohexylcarbonsäure, Benzoesäure, Terephthalsäure, Phthalsäure oder Phenylessigsäure. Besonders bevorzugte Säuren sind aliphatische Mono- und Dicarbonsäuren, insbesondere Essigsäure, Propionsäure sowie C₁₂-C₂₀-Fettsäuren, Bernsteinsäure und Adipinsäure.

Die Menge an zugesetzter Säure pro kg Cyclododecatrien liegt im Allgemeinen bei 0,001 bis 100 g, bevorzugt bei 0,01 bis 50 g und besonders bevorzugt bei 0,05 bis 25 g.

Bei der in situ-Herstellung des Katalysators wird besonders bevorzugt noch mindestens eine CO-Quelle zugesetzt. Diese kann CO selbst sein. Weitere mögliche CO-Quellen sind beispielsweise Formaldehyd, Methanol, Ethanol oder andere geeignete primäre Alkohole wie beispielsweise Benzylalkohol oder Diole oder Polyole mit mindestens einer primären Alkoholgruppe wie beispielsweise Ethylenglykol, Propylenglykol oder Glycerin.

Die Partialhydrierung findet im erfindungsgemäßen Verfahren im Allgemeinen bei Temperaturen im Bereich von 50 bis 300 °C, bevorzugt im Bereich von 80 bis 250 °C und besonders bevorzugt im Bereich von 100 bis 200 °C statt. Die Reaktionsdrücke liegen dabei im Bereich von 1 bis 300 bar, bevorzugt im Bereich von 1 bis 200 bar und besonders bevorzugt im Bereich von 1 bis 100 bar.

Die Reaktionszeiten pro Ansatz in Batch-Fahrweise beziehungsweise die Verweilzeiten bei kontinuierlicher Verfahrensführung liegen im Allgemeinen im Bereich von 0,5 bis 48 h. Sie richten sich im Wesentlichen nach den Ansatzgrößen und den Möglichkeiten, Energie zuführen beziehungsweise abführen zu können. Durch den obenstehend beschriebenen Carbonsäurezusatz ist es nicht kritisch, wenn der Reaktionsansatz länger als erforderlich unter Reaktionsbedingungen gehandhabt wird. Dadurch sind eine erheblich vereinfachte Reaktionsführung und Reaktionskontrolle möglich.

Die bevorzugte Verfahrensführung der Partialhydrierung ist die kontinuierliche Fahrweise. Als Reaktoren sind beispielsweise gerührte oder durch Pumpen durchmischte Reaktoren bevorzugt, wobei das Einbringen von Wasserstoff möglichst effizient sein sollte. Dies kann z.B. durch Wellenbrecher in gerührten Systemen oder durch Stromstörer allgemein erreicht werden.

Gemäß einer unter anderem bevorzugten Ausführungsform der vorliegenden Erfindung wird dort, wo die Hydrierung stattfindet, gleichzeitig die freiwerdende Wärme abgeführt und damit beispielsweise Dampf erzeugt. Diese Verfahrensführung wird beispielsweise bevorzugt in mindestens einem Rohrbündelreaktor durchgeführt. Werden Rohrreaktorsysteme benutzt, so ist es vorteilhaft, beispielsweise durch geeignete Einbauten das Einmischen von Wasserstoff zu beschleunigen, wie es beispielsweise in gepackten Blasensäulen üblich ist.

Zur Vervollständigung des Umsatzes ist es im Rahmen der vorliegenden Erfindung möglich, mindestens zwei Reaktoren hintereinander zu betreiben. Dabei kann beispielsweise ein erster Reaktor stark durchmischt sein, was beispielsweise durch Produktrückführung mittels Pumpe erreicht werden kann, während ein zweiter und gegebenenfalls ein dritter Reaktor lediglich durchströmt werden, wobei gegebenenfalls Wasserstoff zugegeben werden kann. Gemäß einer bevorzugten Ausgestaltung dieser speziellen Verfahrensführung wird im ersten Reaktor ein Umsatz im Bereich von 80 bis 98 % erzielt, während der oder die Nachreaktoren den Restumsatz gewährleisten.

Beim Anfahren der Hydrierung ist es insbesondere bevorzugt, das Edukt Cyclododecatrien nicht oder nicht pur zusammen mit Katalysator und/oder Katalysatorprecursor vorzulegen, da es zu unerwünschten exothermen Reaktionen kommen kann. Im Allgemeinen kann mindestens ein geeignetes Lösungs- oder Verdünnungsmittel zugegeben werden. Als solche sind etwa Cyclododecan, Cyclododecen, gesättigte aliphatische Kohlenwasserstoffe, aromatische Kohlenwasserstoffe oder Gemische aus zwei oder mehr davon zu nennen. Gemäß einer bevorzugten Ausführungsform werden Cyclododecen oder Cyclododecan oder eine Mischung aus Cyclododecen und Cyclododecan oder eine Mischung aus Cyclododecen und Cyclododecatrien oder eine Mischung aus Cyclododecan und Cyclododecatrien oder eine Mischung aus Cyclododecen, Cyclododecan und Cyclododecatrien vorgelegt. Während der Gehalt der entsprechenden Mischungen an Cyclododecatrien im Allgemeinen unkritisch ist, liegt er in kontinuierlichen Verfahren vorzugsweise im Bereich von bis zu 30 Gew.-%, besonderes bevorzugt bei bis zu 25 Gew.-% und insbesondere bevorzugt bei bis zu 20 Gew.-%.

Demgemäß beschreibt die vorliegende Erfindung auch ein wie oben beschriebenes Verfahren, das dadurch gekennzeichnet ist, dass beim Anfahren der Partialhydrierung eine Mischung aus Cyclododecan und/oder Cyclododecen zusammen mit Cyclododecatrien vorgelegt wird, wobei der Gehalt dieser Mischung an Cyclododecatrien im Bereich von bis zu 30 Gew.-% liegt.

Das Produkt, das aus der erfindungsgemäßen Partialhydrierung erhalten wird, stellt im Allgemeinen ein Gemisch dar. Gemäß einer bevorzugten Ausführungsform enthält dieses Gemisch Cyclododecen in einem Bereich von 92 bis 99,9 Gew.-%, weiter bevorzugt im Bereich von 94 bis 99 Gew.-% und insbesondere bevorzugt im Bereich von 96 bis 98 Gew.-%, jeweils bezogen auf das Gesamtgewicht des Produktgemisches.

Im Allgemeinen fällt das Cyclododecen als Gemisch aus cis- und trans-Isomer an. Im Allgemeinen liegt das Molverhältnis von cis-Isomer zu trans-Isomer im Bereich von 0,4:1 bis 2,0:1.

Neben Cyclododecen enthält das Produktgemisch im Allgemeinen Cyclododecan in einem Bereich von 0,1 bis 8 Gew.-%, bevorzugt in einem Bereich von 0,3 bis 7 Gew.-% und besonders bevorzugt in einem Bereich von 0,5 bis 6,5 Gew.-%, jeweils bezogen auf das Gesamtgewicht des Produktgemisches.

Neben Cyclododecen und Cyclododecan kann das Produktgemisch Spuren von Cyclododecadienen und/oder nicht umgesetztem Cyclododecatrien und/oder Katalysator enthalten. Das erfindungsgemäße Verfahren kann prinzipiell so geführt werden, dass das eingesetzte Cyclododecatrien vollständig zu Cyclododecen umgesetzt wird. Im Allgemeinen enthält das Produktgemisch das nicht umgesetzte Edukt Cyclododecatrien in einer Menge von weniger als 0,5 Gew.-%, bevorzugt von weniger als 0,25 Gew.-% und insbesondere bevorzugt von weniger als 0,1 Gew.-%, jeweils bezogen auf das Gesamtgewicht des Produktgemisches.

Sollte dies gewünscht sein, kann nicht umgesetztes Cyclododecatrien durch mindestens eine geeignete Methode wie beispielsweise bevorzugt mindestens eine destillative Maßnahme aus dem Produktgemisch abgetrennt und in das Verfahren rückgeführt werden. Besonders bevorzugt ist es im erfindungsgemäßen Verfahren, aufgrund des sehr hohen Umsatzes an Cyclododecatrien dieses aus dem Produktgemisch aus der Partialhydrierung nicht abzutrennen und Spuren von Cyclododecatrien zusammen mit dem Cyclododecen der Oxidation mit Distickstoffmonoxid gemäß Schritt C zuzuführen.

Gemäß einer bevorzugten Ausführungsform des erfindungsgemäßen Verfahrens wird der für die Partialhydrierung eingesetzte mindestens eine Katalysator aus dem Produktgemisch der Partialhydrierung abgetrennt. Diese Abtrennung kann in Abhängigkeit von eingesetztem Katalysator gemäß jeder geeigneten Verfahrensführung erfolgen.

Wird als Katalysator bei der Partialhydrierung beispielsweise ein heterogener Katalysator als Suspensionskatalysator eingesetzt, so wird dieser im Rahmen der vorliegenden Erfindung bevorzugt durch mindestens einen Filtrationsschritt abgetrennt. Der derart abgetrennte Katalysator kann im Folgenden entweder in das Verfahren rückgeführt werden oder in einem anderen Verfahren eingesetzt werden, verworfen werden oder aufgearbeitet werden, beispielsweise, um das im Katalysator enthaltene mindestens eine Metall zurück zu gewinnen.

Wird als Katalysator bei der Partialhydrierung beispielsweise ein homogener Katalysator eingesetzt, so wird dieser im Rahmen der vorliegenden Erfindung bevorzugt durch mindestens einen Destillationsschritt abgetrennt. Im Rahmen dieser Destillation können eine oder zwei oder mehr Destillationskolonnen verwendet werden.

In der mindestens einen Destillationskolonne wird das Produktgemisch aus der Partialhydrierung in mindestens 2 Fraktionen aufgetrennt. Die Schwersiederfraktion enthält dabei im Wesentlichen die gesamte Menge des eingesetzten homogenen Hydrierkatalysators. Der derart abgetrennte Katalysator kann, gegebenenfalls nach mindestens einem geeigneten Regenerationsschritt, im Folgenden entweder in das Verfahren rückgeführt werden, verworfen werden oder aufgearbeitet werden, beispielsweise, um das im Katalysator enthaltene mindestens eine Metall zurückzugewinnen. Auch der Einsatz des abgetrennten Katalysators in einem anderen Verfahren ist möglich.

Gemäß einer besonders bevorzugten Ausführungsform des erfindungsgemäßen Verfahrens wird ein Teil des derart abgetrennten homogenen Hydrierkatalysators in das Verfahren rückgeführt und der Rest des abgetrennten Katalysators aus dem Verfahren ausgeschleust.

Die Hauptfraktion aus der obenstehend genannten destillativen Aufarbeitung des Produktgemisches aus der Partialhydrierung enthält im Wesentlichen Cyclododecen, mit kleinen Mengen Cyclododecan und gegebenenfalls Spuren an Cyclododecadienen, wie dies bereits obenstehend beschrieben ist.

Gemäß einer bevorzugten Ausführungsform des erfindungsgemäßen Verfahrens wird diese Hauptfraktion der Oxidation mit Distickstoffmonoxid gemäß Schritt C zugeführt.

Ebenso ist es möglich, vor der Zuführung zur Oxidation in mindestens einem geeigneten Destillationsschritt Leichtsieder aus der Hauptfraktion abzutrennen.

Gemäß einer weiteren bevorzugten Ausführungsform des erfindungsgemäßen Verfahrens wird der für die Partialhydrierung eingesetzte mindestens eine Katalysator aus dem Produktgemisch der Partialhydrierung nicht abgetrennt. Diese Ausführungsform ist besonders bevorzugt, wenn für die Hydrierung ein homogener Katalysator eingesetzt wird. Weiter bevorzugt wird in diesem Fall das Produktgemisch aus der Partialhydrierung nicht aufgearbeitet und direkt der Oxidation mit Distickstoffmonoxid gemäß Schritt C zugeführt.

Wie bereits obenstehend beschrieben, wird gemäß einer bevorzugten Ausführungsform im Rahmen der Oxidation des Cyclododecens mit Distickstoffmonoxid gemäß Schritt C ein geeigneter Katalysator eingesetzt, der in der Lage ist, die Gleichgewichtseinstellung zwischen cis- und trans-Isomer des Cyclododecens zu katalysieren.

Gemäß einer besonders bevorzugten Ausführungsform wird als Katalysator für diese Gleichgewichtseinstellung der gleiche Katalysator verwendet wie für die Partialhydrierung des Cyclododecatriens.

Demgemäß betrifft die vorliegende Erfindung auch ein wie oben beschriebenes Verfahren, das dadurch gekennzeichnet ist, dass die Hydrierung von Cyclododecatrien zu Cyclododecen und die Umsetzung von Cyclododecen zu Cyclododecanon mit Distickstoffmonoxid gemäß Schritt C in Anwesenheit des gleichen Katalysators erfolgen.

Einen erheblichen verfahrenstechnischen Vorteil des erfindungsgemäßen Verfahrens stellt die Tatsache dar, dass bei der Verwendung des gleichen homogenen Katalysators bei Partialhydrierung und Oxidation mit Distickstoffmonoxid der Katalysator aus dem Produktgemisch der Partialhydrierung nicht abgetrennt werden muss, sondern dieses Gemisch ohne aufwändige destillative Abtrennung des Katalysators direkt der Oxidation mit Distickstoffmonoxid zugeführt werden kann.

Demgemäß betrifft die vorliegende Erfindung auch ein wie oben beschriebenes Verfahren, das dadurch gekennzeichnet ist, dass ein aus der Hydrierung von Cyclododecatrien zu Cyclododecen in Anwesenheit eines homogenen Katalysators resultierendes Gemisch, enthaltend Cyclododecen und homogenen Katalysator, als Edukt für die Umsetzung mit Distickstoffmonoxid eingesetzt wird.

Im Rahmen der vorliegenden Erfindung ist es weiter möglich, aus dem Produktgemisch der Partialhydrierung nur einen Teil des Katalysators abzutrennen und das resultierende Gemisch, enthaltend Cyclododecen und den restlichen Teil des Katalysators, der Oxidation mit Distickstoffmonoxid zuzuführen. In diesem Fall kann gegebenenfalls mindestens ein weiterer Katalysator bei der Oxidation mit Distickstoffmonoxid zugesetzt werden. Weiter ist es möglich, den Katalysator aus dem Produktgemisch der Partialhydrierung nicht abzutrennen und bei der Oxidation mit Distickstoffmonoxid den gleichen und/oder mindestens einen weiteren Katalysator zuzusetzen.

Das oben beschriebene erfindungsgemäße Verfahren zur Herstellung von Cyclododecanon bietet unter anderem den Vorteil, dass Cyclododecanon in wenigen Schritten und gleichzeitig mit hoher Selektivität erhalten wird. Ein weiterer erheblicher Vorteil ist die Tatsache, dass als ein Edukt für das erfindungsgemäße Verfahren Distickstoffmonoxid enthaltende Abgase aus bevorzugt industriellen Anlagen eingesetzt werden können, die einerseits ohne großen Aufwand verfügbar sind, die andererseits die Integration des erfindungsgemäßen Verfahrens in einen bestehenden Anlagenverbund ermöglichen, wodurch der Transportweg für das Edukt minimal gehalten werden kann, und die weiterhin, als potentielle Klimagase, nicht einer besonderen Behandlung zur Entsorgung zugeführt werden müssen, sondern direkt in ein Wertprodukt fließen.

Das erfindungsgemäß hergestellte Cyclododecanon kann beispielsweise besonders bevorzugt zur Herstellung von Dodecandicarbonsäure und/oder Laurinlactam und/oder daraus abgeleiteten Polymeren wie beispielsweise Polyamiden wie Nylon 12 oder Nylon 6.12 eingesetzt werden.

Soweit die vorliegende Erfindung die Herstellung eines Ketons umfassend das Inkontaktbringen des erfindungsgemäß gereinigten Gasgemischs enthaltend Distickstoffmonoxid mit Cyclododecatrien betrifft, wird in Schritt C des erfindungsgemäßen Verfahrens Cyclododecatrien eingesetzt. Es wird ein Gemisch enthaltend Cyclododecadienon erhalten. Im folgenden werden bevorzugte Verfahrensbedingungen dieser Ausführungsform beschrieben.

Das erhaltene Cyclododecadienon kann vorzugsweise durch Hydrierung zu Cyclododecanon umgesetzt werden.

Die Herstellung von Cyclododeca-4,8-dienon aus 1,5,9-Cyclododecatrien war bislang lediglich über eine zweistufige Synthese möglich, bei der in einem ersten Schritt 1,5,9-Cyclododecatrien zu 1,2-Epoxycyclododeca-5,9-dien epoxidiert wurde und in einem zweiten Schritt das Epoxid katalytisch zu Cyclododeca-4,8-dienon umgelagert wurde. Dieses Verfahren ist beispielsweise in der US 3,063,986 beschriebene. Dieses herkömmliche Verfahren weist zwei entscheidende Nachteile auf: Zum einen ist es schwierig, eine selektive Mono-Epoxidation zu erreichen. Zum anderen weist das Verfahren zwingend eine Zweistufigkeit auf.

Demgegenüber bietet die oben beschriebene, im Rahmen des erfindungsgemäßen Verfahrens bevorzugte Ausführungsform die Möglichkeit, ausgehend von 1,5,9-Cyclododecatrien in einem einzigen Schritt bei hoher Selektivität zu Cyclododeca-4,8-dienon zu gelangen.

Das erfindungsgemäße Gesamtverfahren zur Herstellung von Cyclododecanon umfasst demgemäß Schritte A1, A2, B und C, wobei in Schritt C Cyclododecatrien mit Distickstoffmonoxid unter Erhalt von Cyclododecadienon umgesetzt wird und in einem weiteren Schritt das Cyclododecadienon unter Erhalt von Cyclododecanon hydriert wird.

Gegenüber dem oben beschriebenen gängigen Verfahren, das zwingend die vier Schritte
- Vollständige Hydrierung von Cyclododecatrien zu Cyclododecan;
- Luftoxidation des Cyclododecans in Anwesenheit von Borsäure zu Cyclododecylborat;
- Hydrolyse des Borates zu Cyclododecanol;
- Dehydrierung des Cyclododecanols unter Erhalt von Cyclododecanon
umfasst, zeichnet sich das erfindungsgemäße Verfahren zur Herstellung von Cyclododecanon demgemäß unter anderem dadurch aus, dass ausgehend vom gleichen Edukt, Cyclododecatrien, das Produkt Cyclododecanon unter Einsparung von zwei Reaktionsstufen hergestellt werden kann und die Anzahl der Reaktionsstufen damit halbiert wird.

Im Rahmen der erfindungsgemäßen Umsetzung des Cyclododecatriens mit Distickstoffmonoxid gemäß Schritt C kann mindestens ein geeignetes Lösungsmittel oder Verdünnungsmittel eingesetzt werden. Als solche sind unter anderem Cyclododecan oder Cyclododecanon oder gesättigte aliphatische oder aromatische, gegebenenfalls alkylsubstituierte Kohlenwasserstoffe zu nennen, wobei im Wesentlichen alle gängigen Lösungs- und/oder Verdünnungsmittel geeignet sind unter der Maßgabe, dass sie weder eine C-C-Doppelbindung noch eine C-C-Dreifachbindung noch eine Aldehydgruppe aufweisen.

Im Allgemeinen ist bei der erfindungsgemäßen Umsetzung mit Distickstoffmonoxid gemäß Schritt C der Zusatz eines Lösungsmittels oder Verdünnungsmittel nicht notwendig.

Generell existieren hinsichtlich der Reaktionsbedingungen der Umsetzung des Cyclododecatriens gemäß Schritt C keine besonderen Beschränkungen, solange gewährleistet ist, dass aus der Umsetzung Cyclododeca-4,8-dienon erhalten wird.

Die Temperaturen bei der Umsetzung von Cyclododecatrien mit Distickstoffmonoxid liegen bevorzugt im Bereich von 140 bis 350 °C, weiter bevorzugt im Bereich von 180 bis 320 °C und besonders bevorzugt im Bereich von 200 bis 300 °C.

Im Rahmen des erfindungsgemäßen Verfahrens ist es möglich, die Reaktion gemäß Schritt C bei zwei oder mehr Temperaturen beziehungsweise in zwei oder mehr Temperaturbereichen durchzuführen, die jeweils in den oben angegebenen Grenzen liegen. Temperaturänderungen im Laufe der Umsetzung können kontinuierlich oder auch diskontinuierlich vollzogen werden.

Die Drücke bei der Umsetzung von Cyclododecatrien mit Distickstoffmonoxid gemäß Schritt C liegen bevorzugt höher als der Eigendruck des Edukt- bzw. Produktgemisches bei der gewählten Reaktionstemperatur oder den gewählten Reaktionstemperaturen. Die Drücke liegen bevorzugt im Bereich von 1 bis 1000 bar, weiter bevorzugt im Bereich von 40 bis 300 bar und besonders bevorzugt im Bereich von 50 bis 200 bar.

Im Rahmen des erfindungsgemäßen Verfahrens ist es möglich, die Reaktion bei zwei oder mehr Drücken beziehungsweise in zwei oder mehr Druckbereichen durchzuführen, die jeweils in den oben angegebenen Grenzen liegen. Druckänderungen im Laufe der Umsetzung können kontinuierlich oder auch diskontinuierlich vollzogen werden.

Demgemäß betrifft die vorliegende Erfindung auch ein wie oben beschriebenes Verfahren, das dadurch gekennzeichnet ist, dass die Umsetzung gemäß Schritt C bei einer Temperatur im Bereich von 140 bis 350 °C und einem Druck im Bereich von 1 bis 1000 bar durchgeführt wird.

Hinsichtlich der für die Umsetzung gemäß Schritt C einsetzbaren Reaktoren existieren keine besonderen Beschränkungen. Insbesondere kann die Umsetzung in Batch-Fahrweise oder in kontinuierlicher Fahrweise erfolgen. Demgemäß können beispielsweise als Reaktoren mindestens ein CSTR (Continuous Stirred Tank Reactor) mit mindestens einem internen und/oder mindestens einem externen Wärmetauscher, mindestens ein Rohrreaktor oder mindestens ein Schlaufenreaktor eingesetzt werden. Ebenso ist es möglich, mindestens einen dieser Reaktoren so auszugestalten, dass er mindestens zwei unterschiedliche Zonen aufweist. Solche Zonen können sich beispielsweise in Reaktionsbedingungen wie beispielsweise der Temperatur oder dem Druck und/oder in der Geometrie der Zone wie beispielsweise dem Volumen oder dem Querschnitt unterscheiden. Wird die Umsetzung in zwei oder mehr Reaktoren durchgeführt, können zwei oder mehr gleiche Reaktortypen oder mindestens zwei verschiedene Reaktortypen eingesetzt werden.

Gemäß einer bevorzugten Ausführungsform wird die erfindungsgemäße Umsetzung mit Distickstoffmonoxid gemäß Schritt C in einem einzigen Reaktor durchgeführt. Beispielsweise bevorzugt ist die Umsetzung in kontinuierlicher Fahrweise.

Die Verweilzeit des Reaktionsgutes in dem mindestens einen Reaktor liegt im allgemeinen im Bereich von bis zu 20 h, bevorzugt im Bereich von 0,1 bis 20 Stunden, weiter bevorzugt im Bereich von 0,2 bis 15 Stunden und besonders bevorzugt im Bereich von 0,25 bis 10 h.

Im Feed, der der Umsetzung von Distickstoffmonoxid mit Cyclododecatrien gemäß Schritt C zugeführt wird, liegt das Molverhältnis von Distickstoffmonoxid zu Cyclododecatrien im Allgemeinen im Bereich von 0,05 bis 4, bevorzugt im Bereich von 0,06 bis 1, weiter bevorzugt im Bereich von 0,07 bis 0,5 und besonders bevorzugt im Bereich von 0,1 bis 0,4.

Gemäß einer besonders bevorzugten Ausführungsform wird das erfindungsgemäße Verfahren so durchgeführt, dass bei einer sehr hohen Selektivität bezüglich Cyclododecadienon ein Umsatz von Cyclododecatrien im Bereich von bis zu 50 %, bevorzugt im Bereich von 5 bis 30 % und insbesondere bevorzugt im Bereich von 10 bis 20 % erreicht wird. Dabei liegt die Selektivität, bezogen auf Cyclododecadienon, im Allgemeinen bei mindestens 90 %, bevorzugt bei mindestens 92,5 % und besonders bevorzugt bei mindestens 95 %.

Demgemäß betrifft die vorliegende Erfindung auch ein wie oben beschriebenes Verfahren, das dadurch gekennzeichnet ist, dass die Umsetzung von Distickstoffmonoxid mit Cyclododecatrien gemäß Schritt C bei einer Selektivität, bezogen auf Cyclododecadienon, von mindestens 90 % einen Umsatz von Cyclododecatrien im Bereich von 1 bis 80%, bevorzugt im Bereich von 5 bis 30 % aufweist.

Im Rahmen der vorliegenden Erfindung kann in Schritt C grundsätzlich jedes Cyclododecatrien oder jedes Gemisch aus zwei und mehr unterschiedlichen Cyclododecatrienen mit dem erfindungsgemäß gereinigten Gasgemisch enthaltend Distickstoffmonoxid umgesetzt werden. Unter anderem sind hierbei beispielsweise 1,5,9-Cyclododecatriene, beispielsweise cis,trans,trans-1,5,9-Cyclododecatrien oder cis,cis,trans-1,5,9-Cyclododecatrien oder all-trans-1,5,9-Cyclododecatrien, zu nennen.

Gemäß einer ganz besonders bevorzugten Ausführungsform des erfindungsgemäßen Verfahrens wird als Cyclododecatrien cis,trans,trans-1,5,9-Cyclododecatrien eingesetzt.

Demgemäß betrifft die vorliegende Erfindung auch ein wie oben beschriebenes Verfahren, das dadurch gekennzeichnet ist, dass als Cyclododecatrien cis,trans,trans-1,5,9-Cyclododecatrien eingesetzt wird.

Insbesondere beschreibt die vorliegende Erfindung daher auch ein wie oben beschriebenes Verfahren, das dadurch gekennzeichnet ist, dass gemäß Schritt C cis,trans,trans-1,5,9-Cyclododecatrien mit Distickstoffmonoxid unter Erhalt von Cyclododeca-4,8-dienon umgesetzt wird.

Im allgemeinen resultiert aus der erfindungsgemäßen Umsetzung von cis,trans,trans-1,5,9-Cyclododecatrien mit Distickstoffmonoxid gemäß Schritt C ein Cyclododeca-4,8-dienon-Isomerengemisch, das mindestens zwei der Isomere cis,trans-Cyclododeca-4,8-dienon, trans,cis-Cyclododeca-4,8-dienon und trans,trans-Cyclododeca-4,8-dienon enthält. Bevorzugt wird erfindungsgemäß ein Isomerengemisch erhalten, bei dem trans,cis-Isomer und cis,trans-Isomer in etwa in gleichen Mengen gebildet werden und das trans,trans-Isomer im Vergleich zu den beiden anderen Isomeren in nur geringen Mengen gebildet wird. Ein beispielsweise typisches Isomerengemisch weist demgemäß die Isomeren in molaren Verhältnissen von in etwa 1 : 1 : 0,08 auf.

Die erfindungsgemäße Umsetzung mindestens eines Cyclododecatriens, bevorzugt die Umsetzung mindestens eines 1,5,9-Cyclododecatriens und insbesondere bevorzugt die Umsetzung von cis,trans,trans-1,5,9-Cyclododecatrien gemäß Schritt C kann grundsätzlich in Anwesenheit eines Katalysators erfolgen. Gemäß einer besonders bevorzugten Ausführungsform des erfindungsgemäßen Verfahrens wird die Umsetzung mit Distickstoffmonoxid gemäß Schritt C ohne Zusatz eines Katalysators durchgeführt.

Demgemäß beschreibt die vorliegende Erfindung auch ein wie oben beschriebenes Verfahren, das dadurch gekennzeichnet ist, dass die Umsetzung von Cyclododecatrien mit Distickstoffmonoxid gemäß Schritt C ohne Zusatz eines Katalysators durchgeführt wird.

Im Allgemeinen ist bei der erfindungsgemäßen Umsetzung gemäß Schritt C mit Distickstoffmonoxid der Zusatz eines Lösungsmittels oder Verdünnungsmittel nicht notwendig.

Das bevorzugt eingesetzt 1,5,9-Cyclododecatrien kann beispielsweise durch Trimerisierung von reinem 1,3-Butadien hergestellt werden, wie dies beispielsweise in T. Schiffer, G. Oenbrink, "Cyclodecatriene, Cyclooctadiene, and 4-Vinylcyclohexene", Ullmann's Encyclopedia of Industrial Chemistry, 6th Edition (2000), Electronic Release, Wiley VCH beschrieben ist. Im Rahmen dieses Verfahrens entstehen beispielsweise bei der Trimerisierung in Anwesenheit von Ziegler-Katalysatoren cis,trans,trans-1,5,9-Cyclododecatrien, cis,cis,trans-1,5,9-Cyclododecatrien und all-trans-1,5,9-Cyclododecatrien, wie dies beispielsweise in H. Weber et al. "Zur Bildungsweise von cis,trans,trans-Cyclododecatrien-(1.5.9) mittels titanhaltiger Katalysatoren" in: Liebigs Ann. Chem. 681 (1965) S.10-20 beschrieben ist. Während sämtliche dieser Cyclododecatriene im Rahmen des erfindungsgemäßen Verfahrens mittels Distickstofmonoxid einzeln oder als Gemisch aus zwei oder mehr davon oxidiert werden können, ist, wie oben beschrieben, die Umsetzung von cis,trans,trans-1,5,9-Cyclododecatrien im Rahmen des vorliegenden erfindungsgemäßen Verfahrens besonders bevorzugt. Dieses cis,trans,trans-1,5,9-Cyclododecatrien wird besonders bevorzugt gemäß des in dem oben erwähnten Artikel von Weber et al. hergestellt.

Demgemäß beschreibt die vorliegende Erfindung auch ein wie oben beschriebenes Verfahren, das dadurch gekennzeichnet ist, dass das als Edukt eingesetzte Cyclododecatrien durch Trimerisierung von 1,3-Butadien unter Verwendung eines Titan-Katalysators hergestellt wird.

Während grundsätzlich sämtliche geeigneten Titan-Katalysatoren zur Trimerisierung eingesetzt werden können, ist der im Artikel von Weber et al. beschriebene Titantetrachlorid/Ethylaluminiumsesquichlorid-Katalysator besonders bevorzugt.

Das für die Trimerisierung eingesetzte Butadien weist insbesondere bevorzugt einen über Gaschromatographie bestimmten Reinheitsgrad von mindestens 99,6 % und weiter bevorzugt von mindestens 99,65 % auf. Insbesondere bevorzugt enthält das eingesetzte 1,3-Butadien im Rahmen der Nachweisgenauigkeit kein 1,2-Butadien und kein 2-Butin.

Aus dieser bevorzugten Trimerisierung werden im Allgemeinen Gemische erhalten, die mindestens 95 Gew.-%, bevorzugt mindestens 96 Gew.-% und weitere bevorzugt mindestens 97 Gew.-% cis,trans,trans-1,5,9-Cyclododecatrien enthalten. Beispielsweise insbesondere bevorzugt enthalten die Gemische in etwa 98 Gew.-% cis,trans,trans-1,5,9-Cyclododecatrien.

Dieses cis,trans,trans-1,5,9-Cyclododecatrien enthaltende Gemisch kann als solches für die Umsetzung mit Distickstoffmonoxid gemäß Schritt C verwendet werden. Ebenso ist es möglich, über mindestens eine geeignete Methode, beispielsweise bevorzugt über mindestens eine Destillation, das cis,trans,trans-1,5,9-Cyclododecatrien aus dem Gemisch abzutrennen und in der Umsetzung mit Distickstoffmonoxid einzusetzen. Bevorzugt findet eine solche Aufreinigung im Rahmen des erfindungsgemäßen Verfahrens nicht statt.

Hinsichtlich weiterer Details zur Trimerisierung sei auf den Artikel von Weber et al. verwiesen.

Aus der erfindungsgemäßen Umsetzung von Cyclododecatrien mit Distickstoffmonoxid gemäß Schritt C wird im Allgemeinen ein Gemisch erhalten, das Cyclododecadienon, bevorzugt Cyclododeca-4,8-dienon, und gegebenenfalls nicht umgesetztes Edukt und/oder gegebenenfalls mindestens ein Nebenprodukt enthält. Je nach weiterer Verwertung und/oder Aufarbeitung kann aus diesem Gemisch das Cyclododecadienon, bevorzugt das Cyclododeca-4,8-dienon abgetrennt werden. Im Falle, dass das Gemisch Cyclododecadienon und beispielsweise ein Diketon wie Cyclododecendion enthält, ist es möglich, das Cyclododecadienon, bevorzugt das Cyclododeca-4,8-dienon, in einfacher Weise abzutrennen und einem weiteren Verfahrensschritt wie beispielsweise der Partialhydrierung zu Cyclododecenon oder bevorzugt der Hydrierung zu Cyclododecanon zuzuführen.

Aus diesem Gemisch kann das Cyclododeca-4,8-dienon durch mindestens eine geeignete Methode abgetrennt werden. Bevorzugt ist hierbei die destillative Abtrennung. Die Destillation erfolgt hierbei bei einem Druck im Bereich von im Allgemeinen 0,001 bis 2 bar, bevorzugt im Bereich von 0,01 bis 1 bar und insbesondere bevorzugt im Bereich von 0,03 bis 0,5 bar.

Das erfindungsgemäß aus der Umsetzung von Cyclododecatrien mit Distickstoffmonoxid erhaltene Cyclododecadienon, kann einem oder mehreren beliebigen weiteren Verfahren zugeführt werden. Beispielsweise kann die Ketogruppe des Cyclododecadienons einer chemischen Reaktion unterworfen werden. Ebenso kann mindestens eine der C-C-Doppelbindungen einer chemischen Reaktion unterworfen werden. Beispielsweise bevorzugt können mindestens eine C-C-Doppelbindung, bevorzugt beide C-C-Doppelbindungen hydriert werden.

Unabhängig davon, welches Regioisomere von Cyclododecadienon oder welches Gemisch aus mindestens zwei regioisomeren Cyclododecadienonen aus der erfindungsgemäßen Umsetzung mit Distickstoffmonoxid erhalten wird, wird dieses Regioisomere oder dieses Regioisomerengemisch bevorzugt zu Cyclododecanon hydriert.

Gemäß einer besonders bevorzugten Ausführungsform des erfindungsgemäßen Verfahrens wird Cyclododeca-4,8-dienon zu Cyclododecanon hydriert.

Demgemäß betrifft die vorliegende Erfindung auch ein wie oben beschriebenes Verfahren, das dadurch gekennzeichnet ist, dass das aus der Umsetzung von Cyclododecatrien mit Distickstoffmonoxid gemäß Schritt C erhaltene Cyclododecadienon unter Erhalt von Cyclododecanon hydriert wird.

Für die Hydrierung des Cyclododecadienons und insbesondere bevorzugt Cyclododeca-4,8-dienons können sämtliche geeigneten Katalysatoren eingesetzt werden. Insbesondere sind mindestens ein homogener oder mindestens ein heterogener oder sowohl mindestens ein homogener und mindestens ein heterogener Katalysator einsetzbar.

Bevorzugt enthalten die einsetzbaren Katalysatoren mindestens ein Metall aus der 7., der 8., der 9., der 10. oder der 11. Nebengruppe des Periodensystems der Elemente. Weiter bevorzugt enthalten die erfindungsgemäß einsetzbaren Katalysatoren mindestens ein Element, ausgewählt aus der Gruppe bestehend aus Re, Fe, Ru, Co, Rh, Ir, Ni, Pd, Pt, Cu und Au. Insbesondere bevorzugt enthalten die erfindungsgemäß einsetzbaren Katalysatoren mindestens ein Element, ausgewählt aus der Gruppe bestehend aus Fe, Ni, Pd, Pt und Cu. Besonders bevorzugt enthalten die erfindungsgemäß einsetzbaren Katalysatoren Pd.

Im erfindungsgemäßen Verfahren bevorzugt eingesetzte homogene Katalysatoren enthalten mindestens ein Element der 8., 9. oder 10. Nebengruppe. Weiter bevorzugt sind homogenen Katalysatoren, die Ru, Rh, Ir und/oder Ni enthalten. Beispielsweise sind hierbei etwa RhCl(TTP)₃ oder Ru₄H₄(CO)₁₂ zu nennen. Besonders bevorzugt sind solche homogenen Katalysatoren, die Ru enthalten. Beispielsweise werden homogene Katalysatoren eingesetzt, wie sie in der US 5,180,870, US 5,321,176, US 5,177,278, US 3,804,914, US 5,210,349 US 5,128,296, US B 316,917 und in D.R. Fahey in J. Org. Chem. 38 (1973) S. 80-87 beschrieben sind. Solche Katalysatoren sind etwa (TPP)₂(CO)₃Ru, [Ru(CO)₄]₃, (TPP)₂Ru(CO)₂Cl₂, (TPP)₃(CO)RuH₂, (TPP)₂(CO)₂RuH₂, (TPP)₂(CO)₂RuClH oder (TPP)₃(CO)RuCl₂.

Insbesondere bevorzugt wird im Rahmen des erfindungsgemäßen Verfahrens mindestens ein heterogener Katalysator eingesetzt, wobei mindestens eines der obenstehend genannten Metalle als Metall als solches, als Raney-Katalysator und/oder aufgebracht auf einen üblichen Träger eingesetzt werden kann. Bevorzugte Trägermaterialien sind etwa Aktivkohlen oder Oxide wie beispielsweise Aluminiumoxide, Siliciumoxide, Titanoxide oder Zirkonoxide. Ebenso sind unter anderem als Trägermaterialien Bentonite zu nennen. Werden zwei oder mehr Metalle eingesetzt, so können diese separat oder als Legierung vorliegen. Hierbei ist es möglich, mindestens ein Metall als solches und mindestens ein anderes Metall als Raney-Katalysator oder mindestens ein Metall als solches und mindestens ein anderes Metall, aufgebracht auf mindestens einen Träger, oder mindestens ein Metall als Raney-Katalysator und mindestens ein anderes Metall, aufgebracht auf mindestens einen Träger, oder mindestens ein Metall als solches und mindestens ein anderes Metall als Raney-Katalysator und mindestens ein anderes Metall, aufgebracht auf mindestens einen Träger, einzusetzen.

Die im erfindungsgemäßen Verfahren eingesetzten Katalysatoren können beispielsweise auch so genannte Fällungskatalysatoren sein. Solche Katalysatoren können hergestellt werden, indem man ihre katalytisch aktiven Komponenten aus deren Salzlösungen, insbesondere aus den Lösungen von deren Nitraten und/oder Acetaten, beispielsweise durch Zugabe von Lösungen von Alkalimetall- und/oder Erdalkalimetallhydroxid- und/oder Carbonat-Lösungen, beispielsweise schwerlöslichen Hydroxiden, Oxidhydraten, basischen Salze oder Carbonaten ausfällt, die erhaltenen Niederschläge anschließend trocknet und diese dann durch Calcinierung bei im allgemeinen 300 bis 700 °C, insbesondere 400 bis 600 °C in die entsprechenden Oxide, Mischoxide und/oder gemischtvalentigen Oxide umwandelt, die durch eine Behandlung mit Wasserstoff oder mit Wasserstoff enthaltenden Gasen im Bereich von im Allgemeinen 50 - 700 °C, insbesondere 100 bis 400 °C zu den betreffenden Metallen und/oder oxidischen Verbindungen niederer Oxidationsstufe reduziert und in die eigentliche katalytisch aktive Form überführt werden. Dabei wird in der Regel so lange reduziert, bis kein Wasser mehr gebildet wird. Bei der Herstellung von Fällungskatalysatoren, die ein Trägermaterial enthalten, kann die Fällung der katalytisch aktiven Komponenten in Gegenwart des betreffenden Trägermaterials erfolgen. Die katalytisch aktiven Komponenten können vorteilhaft gleichzeitig mit dem Trägermaterial aus den betreffenden Salzlösungen gefällt werden.

Bevorzugt werden im erfindungsgemäßen Verfahren Hydrierkatalysatoren eingesetzt, welche die die Hydrierung katalysierenden Metalle oder Metallverbindungen auf einem Trägermaterial abgeschieden enthalten.

Außer den oben genannten Fällungskatalysatoren, die außer den katalytisch aktiven Komponenten noch zusätzlich ein Trägermaterial enthalten, eignen sich für das erfindungsgemäße Verfahren im Allgemeinen solche Trägermaterialien, bei denen die katalytischhydrierend wirkende Komponente beispielsweise durch Imprägnierung auf ein Trägermaterial aufgebracht worden sind.

Die Art des Aufbringens des katalytisch aktiven Metalls auf den Träger ist in der Regel nicht kritisch und kann auf verschiedenerlei Art und Weise bewerkstelligt werden. Die katalytisch aktiven Metalle können auf diesen Trägermaterialien beispielsweise durch Tränkung mit Lösungen oder Suspensionen der Salze oder Oxide der betreffenden Elemente, Trocknung und anschließender Reduktion der Metallverbindungen zu den betreffenden Metallen oder Verbindungen niederer Oxidationsstufe mittels eines Reduktionsmittels, vorzugsweise mit Wasserstoff oder komplexen Hydriden, aufgebracht werden. Eine andere Möglichkeit zur Aufbringung der katalytisch aktiven Metalle auf diese Träger besteht darin, die Träger mit Lösungen thermisch leicht zersetzbarer Salze, beispielsweise mit Nitraten oder thermisch leicht zersetzbaren Komplexverbindungen, beispielsweise Carbonyl- oder Hydrido-Komplexen der katalytisch aktiven Metalle, zu imprägnieren und den so getränkten Träger zur thermischen Zersetzung der adsorbierten Metallverbindungen auf Temperaturen im Bereich von 300 bis 600 °C zu erhitzen. Diese thermische Zersetzung wird vorzugsweise unter einer Schutzgasatmosphäre vorgenommen. Geeignete Schutzgase sind beispielsweise Stickstoff, Kohlendioxid, Wasserstoff oder die Edelgase. Weiterhin können die katalytisch aktiven Metalle auf dem Katalysatorträger durch Aufdampfen oder durch Flammspritzen abgeschieden werden. Der Gehalt dieser Trägerkatalysatoren an den katalytisch aktiven Metallen ist prinzipiell für das Gelingen des erfindungsgemäßen Verfahrens nicht kritisch. Im Allgemeinen führen höhere Gehalte an katalytisch aktiven Metallen dieser Trägerkatalysatoren zu höheren Raum-Zeit-Umsätzen als niedrigere Gehalte. Im Allgemeinen werden Trägerkatalysatoren verwendet, deren Gehalt an katalytisch aktiven Metallen im Bereich von 0,1 bis 90 Gew.-%, vorzugsweise im Bereich von 0,5 bis 40 Gew.-% bezogen auf das Gesamtgewicht des Katalysators, liegt. Da sich diese Gehaltsangaben auf den gesamten Katalysator inklusive Trägermaterial beziehen, die unterschiedlichen Trägermaterialien jedoch sehr unterschiedliche spezifische Gewichte und spezifische Oberflächen haben, ist es auch denkbar, dass diese Angaben auch unter- oder überschritten werden können, ohne dass sich dies nachteilig auf das Ergebnis des erfindungsgemäßen Verfahrens auswirkt. Selbstverständlich können auch mehrere der katalytisch aktiven Metalle auf dem jeweiligen Trägermaterial aufgebracht sein. Weiterhin können die katalytisch aktiven Metalle beispielsweise nach dem Verfahren von DE-OS 25 19 817 oder EP 0 285 420 A1 auf den Träger aufgebracht werden. In den Katalysatoren gemäß den vorgenannten Schriften liegen die katalytisch aktiven Metalle als Legierung vor, die durch thermische Behandlung und/oder Reduktion der z.B. durch Tränken mit einem Salz oder Komplex der zuvor genannten Metalle, erzeugt werden.

Sowohl die Aktivierung der Fällungskatalysatoren als auch der Trägerkatalysatoren kann auch in situ zu Beginn der Reaktion durch den anwesenden Wasserstoff erfolgen. Bevorzugt werden diese Katalysatoren vor ihrer Verwendung separat aktiviert.

Als Trägermaterialien können im Allgemeinen die Oxide des Aluminiums und Titans, Zirkoniumdioxid, Siliciumdioxid, Tonerden wie beispielsweise Montmorillonite, Silikate wie beispielsweise Magnesium- oder Aluminiumsilikate, Zeolithe wie beispielsweise der Strukturtypen ZSM-5 oder ZSM-10, oder Aktivkohle verwendet werden. Bevorzugte Trägermaterialien sind Aluminiumoxide, Titandioxide, Siliciumdioxid, Zirkoniumdioxid und Aktivkohle. Selbstverständlich können auch Mischungen verschiedener Trägermaterialien als Träger für im erfindungsgemäßen Verfahren verwendbaren Katalysatoren dienen.

Der mindestens eine heterogene Katalysator kann beispielsweise als Suspensionskatalysator und/oder als Festbettkatalysator eingesetzt werden.

Wird beispielsweise im Rahmen des erfindungsgemäßen Verfahrens die Hydrierung mit mindestens einem Suspensionskatalysator durchgeführt, so wird bevorzugt in mindestens einem Rührreaktor oder in mindestens einer Blasensäule oder in mindestens einer gepackten Blasensäule oder in einer Kombination aus zwei oder mehr gleichen oder unterschiedlichen Reaktoren hydriert.

Der Begriff "unterschiedliche Reaktoren" bezeichnet vorliegend sowohl unterschiedliche Reaktortypen als auch Reaktoren der gleichen Art, die sich beispielsweise durch ihre Geometrie wie beispielsweise ihrem Volumen und/oder ihrem Querschnitt und/oder durch die Hydrierbedingungen in den Reaktoren unterscheiden.

Wird beispielsweise im Rahmen des erfindungsgemäßen Verfahrens die Hydrierung mit mindestens einem fest angeordneten Katalysator durchgeführt, so wird bevorzugt mindestens ein Rohrreaktor wie beispielsweise mindestens ein Schachtreaktor und/oder mindestens ein Rohrbündelreaktor verwendet, wobei ein einzelner Reaktor in Sumpf- oder Rieselfahrweise betrieben werden kann. Bei Verwendung von zwei oder mehr Reaktoren kann mindestens einer in Sumpffahrweise und mindestens einer in Rieselfahrweise betrieben werden.

Gemäß einer bevorzugten Ausführungsform des erfindungsgemäßen Verfahrens wird der für die Hydrierung eingesetzte mindestens eine Katalysator aus dem Produktgemisch der Hydrierung abgetrennt. Diese Abtrennung kann in Abhängigkeit vom eingesetzten Katalysator gemäß jeder geeigneten Verfahrensführung erfolgen.

Wird als Katalysator bei der Hydrierung beispielsweise ein heterogener Katalysator als Suspensionskatalysator eingesetzt, so wird dieser im Rahmen der vorliegenden Erfindung bevorzugt durch mindestens einen Filtrationsschritt abgetrennt. Der derart abgetrennte Katalysator kann in die Hydrierung rückgeführt werden oder mindestens einem beliebigen anderen Verfahren zugeführt werden. Ebenso ist es möglich, den Katalysator aufzuarbeiten, um beispielsweise das in dem Katalysator enthaltene Metall zurückzugewinnen.

Wird als Katalysator bei der Hydrierung beispielsweise ein homogener Katalysator eingesetzt, so wird dieser im Rahmen der vorliegenden Erfindung bevorzugt durch mindestens einen Destillationsschritt abgetrennt. Im Rahmen dieser Destillation können eine oder zwei oder mehr Destillationskolonnen verwendet werden. Der derart abgetrennte Katalysator kann in die Hydrierung rückgeführt werden oder mindestens einem beliebigen anderen Verfahren zugeführt werden. Ebenso ist es möglich, den Katalysator aufzuarbeiten, um beispielsweise das in dem Katalysator enthaltene Metall zurückzugewinnen.

Vor dem Einsatz in einem beliebigen Verfahren wie beispielsweise vor der Rückführung in das erfmdungsgemäße Verfahren kann sowohl der mindestens eine homogene als auch der mindestens eine heterogene Katalysator, sollte dies erforderlich sein, durch mindestens ein geeignetes Verfahren regeneriert werden.

Die Wärmeabfuhr kann bei dem erfindungsgemäß verwendeten Reaktor intern beispielsweise über Kühlschlangen und/oder extern beispielsweise über mindestens einen Wärmetauscher erfolgen. Wird beispielsweise bevorzugt mindestens ein Rohrreaktor zur Hydrierung eingesetzt, so wird bevorzugt die Reaktion über äußeren Umlauf gefahren, in dem die Wärmeabfuhr integriert ist.

Wird gemäß einer bevorzugten Ausführungsform des erfindungsgemäßen Verfahrens die Hydrierung kontinuierlich durchgeführt, werden weiter bevorzugt mindestens zwei Reaktoren, weiter bevorzugt mindestens zwei Rohrreaktoren, weiter bevorzugt mindestens zwei seriell gekoppelte Rohrreaktoren und insbesondere bevorzugt zwei seriell gekoppelte Rohrreaktoren eingesetzt. Die Hydrierbedingungen in den eingesetzten Reaktoren können jeweils gleich oder unterschiedlich sein und liegen jeweils in den oben beschriebenen Bereichen.

Wird die Hydrierung an mindestens einem suspendierten Katalysator durchgeführt, liegt die Verweilzeit im Allgemeinen im Bereich von 0,5 bis 50 h, bevorzugt im Bereich von 1 bis 30 h und besonders bevorzugt im Bereich von 1,5 bis 25 h. Dabei ist es unerheblich, ob erfindungsgemäß ein Reaktor oder mindestens 2 in Serie geschaltete Reaktoren eingesetzt werden. Für sämtliche dieser Ausführungsformen liegt die Gesamtverweilzeit in den oben angegebenen Bereichen.

Wird im Rahmen des erfindungsgemäßen Verfahrens die Hydrierung in kontinuierlicher Fahrweise an mindestens einem fest angeordneten Katalysator durchgeführt, so liegt die Verweilzeit im Allgemeinen im Bereich von 0,1 bis 20 h, bevorzugt im Bereich von 0,2 bis 15 h und besonders bevorzugt im Bereich von 0,3 bis 10 h. Dabei ist es unerheblich, ob erfindungsgemäß ein Reaktor oder mindestens 2 in Serie geschaltete Reaktoren eingesetzt werden. Für sämtliche dieser Ausführungsformen liegt die Gesamtverweilzeit in den oben angegebenen Bereichen.

Das Gemisch, das aus dem ersten Rohrreaktor erhalten wird, enthält Cyclododecanon in einem Anteil, bezogen auf den Gesamtgehalt des Gemisches an C₁₂-Komponenten, bevorzugt im Bereich von 50 bis 99,9 Gew.-% und besonders bevorzugt im Bereich von 70 bis 99,5 Gew.-%. Dieses Gemisch wird, gegebenenfalls nach mindestens einer geeigneten Zwischenbehandlung, dem zweiten Rohrreaktor zugeführt. Das Gemisch, das aus dem zweiten Rohrreaktor erhalten wird, enthält Cyclododecanon, in einem Anteil bevorzugt im Bereich von mindestens 99,5 %, weiter bevorzugt im Bereich von 99,9 % und insbesondere bevorzugt von 99,99 Gew.-%.

Der Wasserstoffdruck liegt bei der erfindungsgemäßen Hydrierung im Allgemeinen im Bereich von 1 bis 325 bar, bevorzugt im Bereich von 1,5 bis 200 bar, weiter bevorzugt im Bereich von 2 bis 100 bar und insbesondere bevorzugt im Bereich von 2,5 bis 50 bar.

Die Hydriertemperatur liegt im Allgemeinen im Bereich von 0 bis 250 °C, bevorzugt im Bereich von 20 bis 200 °C weiter bevorzugt im Bereich von 30 bis 180 °C und insbesondere bevorzugt im Bereich von 40 bis 170 °C.

Demgemäß betrifft die vorliegende Erfindung auch ein wie oben beschriebenes Verfahren, das dadurch gekennzeichnet ist, dass die Hydrierung in Anwesenheit eines Hydrierkatalysators, bevorzugt eines heterogenen Hydrierkatalysators, bei einer Temperatur im Bereich von 0 bis 250 °C und einem Druck im Bereich von 1 bis 325 bar durchgeführt wird.

Im Rahmen der erfindungsgemäßen Hydrierung kann mindestens ein geeignetes Lösungsmittel oder Verdünnungsmittel eingesetzt werden. Als solche sind unter anderem Cyclododecanon oder Cyclododecan sowie grundsätzlich sämtliche Lösungs- und Verdünnungsmittel zu nennen, die unter den Hydrierbedingungen nicht hydriert oder anderweitig umgesetzt werden.

Gemäß einer bevorzugten Ausführungsform des erfindungsgemäßen Verfahrens wird die Hydrierung ohne Zusatz eines Lösungs- oder Verdünnungsmittels durchgeführt.

Aus der erfindungsgemäßen Hydrierung wird im Allgemeinen ein Gemisch erhalten, das neben Cyclododecanon gegebenenfalls mindestens ein Nebenprodukt und/oder mindestens ein nicht umgesetztes Edukt und/oder mindestens eine weitere Verbindung enthält, die über beispielsweise ein Gemisch, enthaltend Edukt, der Hydrierung zugeführt wurde. Aus diesem Gemisch kann das Cyclododecanon über mindestens eine geeignete Methode wie beispielsweise bevorzugt über mindestens eine Destillation abgetrennt werden.

Das oben beschriebene erfindungsgemäße Verfahren zur Herstellung von Cyclododecanon bietet unter anderem den Vorteil, dass Cyclododecanon und auch Cyclododecadienon in wenigen Schritten und gleichzeitig mit hoher Selektivität erhalten werden. Ein weiterer erheblicher Vorteil ist die Tatsache, dass als ein Edukt für das erfindungsgemäße Verfahren Distickstoffmonoxid enthaltende Abgase aus bevorzugt industriellen Anlagen eingesetzt werden können, die einerseits ohne großen Aufwand verfügbar sind, die andererseits die Integration des erfindungsgemäßen Verfahrens in einen bestehenden Anlagenverbund ermöglichen, wodurch der Transportweg für das Edukt minimal gehalten werden kann, und die weiterhin, als potentielle Klimagase, nicht einer besonderen Behandlung zur Entsorgung zugeführt werden müssen, sondern direkt in ein Wertprodukt fließen.

Das erfindungsgemäß insbesondere bevorzugt erhaltene und gegebenenfalls aus dem Produktgemisch abgetrennte Cyclododecanon kann beispielsweise besonders bevorzugt zur Herstellung von Dodecandicarbonsäure und/oder Laurinlactam und/oder daraus abgeleiteten Polymeren wie beispielsweise Polyamiden wie Nylon 12 oder Nylon 6.12 eingesetzt werden.

Die vorliegende Erfindung wird durch die folgenden Beispiele illustriert.

### Beispiele:

### Beispiel 1

### Einstufige Absorption mit Tetradecan

Ein Eduktgas (151 mol/h) mit der in Tabelle 1.1 angegebenen Zusammensetzung wird auf 25 bara komprimiert, auf 30°C abgekühlt und am untersten Boden einer Absorptionskolonne (∅= 8 cm, Höhe= 400 cm, mit Packung Kühni Rombopak) zugeführt. Von oben werden 96 kg/h technisches Tetradecan (von der Desorberstufe kommend) zugeführt. Das abgereicherte Abgas am Kopf der Kolonne wird verworfen. Das beladene Lösungsmittel am Sumpf der Absorptionskolonne wird in einen Flash-Behälter (∅= 20 cm, Höhe= 50 cm bei 30°C betrieben) auf 1 bara entspannt. Das Lösungsmittel wird dann in die Absorberstufe zurückgepumpt. Das desorbierte Produktgas (12,9 mol/h) wird analysiert und verworfen. Die Zusammensetzung ist ebenfalls in Tabelle 1.1 angegeben.

**Tabelle 1.1**

| Komponente | Zusammensetzung / Vol.-% | |
|---|---|---|
| | Eduktgas | Produktgas |
| N₂O | 6,3 | 49 |
| O₂ | 4,3 | 2,6 |
| N₂ | 87,7 | 44,8 |
| NOₓ | 0,05 | 0,16 |
| CO₂ | 0,6 | 3,3 |

Die formale Ausbeute an N₂O (definiert als 100×(mol N₂O im Produktgas)/(mol N₂O im Eduktgas)) beträgt 66%.

### Beispiel 2

### Zweistufige Absorption mit Tetradecan

Beispiel 1 wurde wiederholt wobei das Produktgas aus der ersten Desorberstufe 12,9 mol/h) und mit der in Tabelle 1.2 angegebenen Zusammensetzung auf 25 bara komprimiert, auf 30°C abgekühlt und am untersten Boden einer zweiten Absorptionskolonne (∅= 5 cm, Höhe= 400 cm, mit Packung Kühni Rombopack) zugeführt wird. Von oben werden 12 kg/h technisches Tetradecan (von der zweiten Desorberstufe kommend) zugeführt. Das abgereicherte Abgas am Kopf der zweite Absorptionskolonne wird verworfen. Das beladene Lösungsmittel am Sumpf der zweiten Absorptionskolonne wird in einem zweiten Flash-Behälter (∅= 10 cm, Höhe= 40 cm bei 30°C betrieben) auf 1 bara entspannt. Das Lösungsmittel wird zurück in die zweite Absorberstufe gepumpt. Das desorbierte Produktgas aus der zweiten Kolonne (6,8 mol/h) wird dann analysiert. Die Zusammensetzung ist ebenfalls in Tabelle 1.2 angegeben.

**Tabelle 1.2**

| Komponente | Zusammensetzung / Vol.-% | |
|---|---|---|
| | Eduktgas 2. Stufe (=Produktgas 1. Stufe) | Produktgas 2. Stufe |
| N₂O | 49 | 88,5 |
| O₂ | 2,6 | 0,9 |
| N₂ | 44,8 | 1,5 |
| NOₓ | 0,16 | 0,4 |
| CO₂ | 3,3 | 8,5 |

Die formale Ausbeute an N₂O (definiert als 100×(mol N₂O im Produktgas der 2. Stufe)/( mol N₂O im Eduktgas der 1. Stufe)) beträgt 63 %.

### Beispiel 3

### Zweistufige Absorption mit Tetradecan und anschließende Wäsche

Beispiel 2 wurde wiederholt wobei das Produktgas aus der zweiten Desorberstufe (6,8 mol/h) mit der in Tabelle 1.3 angegebenen Zusammensetzung dem untersten Boden einer Waschkolonne (∅= 5 cm, Höhe= 300 cm, mit 10 mm Pallringen) zugeführt wird. Von oben werden 20 kg/h einer wässrige NaHCO₃-Lösung (Konzentration: 40 g/kg) zugeführt. Das Gas am Kopf der Waschkolonne wird dann analysiert. Die Zusammensetzung ist ebenfalls in Tabelle 1.3 angegeben.

**Tabelle 1.3**

| Komponente | Zusammensetzung / Vol.-% | |
|---|---|---|
| | Eduktgas Waschkolonne (=Produktgas 2. Stufe) | Produktgas nach der Waschkolonne |
| N₂O | 88,5 | 87,6 |
| O₂ | 0,9 | 0,9 |
| N₂ | 1,5 | 1,4 |
| NOₓ | 0,4 | 0,1 |
| CO₂ | 8,5 | 9,6 |

Die formale Ausbeute an N₂O (definiert als 100×( mol N₂O im Produktgas nach der Waschkolonne)/(mol N₂O im Eduktgas der 1. Stufe)) beträgt 62 %.

### Beispiel 4

### Zweistufige Absorption mit Tetradecan mit anschließender Wäsche und Verflüssigung

Beispiel 3 wurde wiederholt wobei das Produktgas nach der Waschkolonne (6,7 mol/h) mit der in Tabelle 1.4 angegebenen Zusammensetzung stufenweise auf 25 bara komprimiert und auf -30 °C abgekühlt wird. Das kondensierte Produkt (6,6mol/h) wird dann analysiert. Die Zusammensetzung ist ebenfalls in Tabelle 1.4 angegeben.

**Tabelle 1.4**

| Komponente | Zusammensetzung / Vol.-% | |
|---|---|---|
| | Produktgas nach der Waschkolonne | Flüssiges Produkt |
| N₂O | 87,6 | 89,1 |
| O₂ | 0,9 | 0,1 |
| N₂ | 1,4 | 1,2 |
| NOₓ | 0,1 | 0,1 |
| CO₂ | 9,6 | 8,6 |

Die formale Ausbeute an N₂O (definiert als 100×( mol N₂O im verflüssigten Gas)/(mol N₂O im Eduktgas der 1. Stufe)) beträgt 61 %.

### Beispiel 5

### Zweistufige Absorption mit Nitrobenzol

Ein Eduktgas (150 mol/h) mit der in Tabelle 1.5 angegebenen Zusammensetzung wird auf 25 bar komprimiert, auf 30°C abgekühlt und am untersten Boden einer erste Absorptionskolonne (∅= 8 cm, Höhe= 400 cm, mit Packung Kühni Rombopack) zugeführt. Von oben werden 63 kg/h Nitrobenzol (von der erste Desorberstufe kommend) zugeführt. Das abgereichte Abgas am Kopf der ersten Absorptionskolonne wird verworfen. Das beladene Lösungsmittel am Sumpf der ersten Absorptionskolonne wird in einem Flash-Behälter (∅= 20 cm, Höhe= 50 cm bei 30°C betrieben) auf 1 bar entspannt. Das Lösungsmittel wird dann in die erste Absorberstufe zurückgepumpt. Das desorbierte Produktgas aus der ersten Desorberstufe wird dann auf 25 bar komprimiert, auf 30°C abgekühlt und dem untersten Boden einer zweiten Absorptionskolonne (∅= 5 cm, Höhe= 400 cm, mit Packung Kühni Rombopack) zugeführt. Von oben werden 5 kg/h Nitrobenzol (von der zweiten Desorberstufe kommend) zugeführt. Das abgereicherte Abgas am Kopf der zweiten Absorptionskolonne wird verworfen. Das beladene Lösungsmittel am Sumpf der zweiten Absorptionskolonne wird in einem zweiten Flash-Behälter (∅= 10 cm, Höhe= 40 cm bei 30°C betrieben) auf 1 bar entspannt. Das Lösungsmittel wird zurück in die zweite Absorberstufe gepumpt. Das desorbierte Produktgas aus der zweiten Kolonne (8,4 mol/h) wird dann analysiert. Die Zusammensetzung ist ebenfalls in Tabelle 1.5 angegeben.

**Tabelle 1.5**

| Komponente | Zusammensetzung / Vol.-% | |
|---|---|---|
| | Eduktgas | Produktgas |
| N₂O | 9 | 90 |
| O₂ | 5,1 | 0,1 |
| N₂ | 84,1 | 0,7 |
| NOₓ | 0,05 | 0,2 |
| CO₂ | 0,87 | 8,7 |

Die formale Ausbeute an N₂O (definiert als 100×(mol N₂O im Produktgas der 2. Stufe)/( mol N₂O im Eduktgas der 1. Stufe)) beträgt 56 %.

### Beispiel 6

### Wäsche, zweistufige Absorption mit Nitrobenzol, Hydrogencarbonatwäsche und Verflüssigung

Ein Eduktgas (210 mol/h) mit der in Tabelle 1.6 angegebenen Zusammensetzung wird auf 4 bar komprimiert und zuerst in einer Waschkolonne (∅= 8 cm, Höhe= 400 cm, mit Packung Kühni Rombopack) zu dem 3,3 kg/h Wasser zugefahren werden, bei 40 °C gewaschen. Die gebildete verdünnte Salpetersäure wird zur weiteren Verwertung abgefüllt. Das gewaschene Gas wird dann auf 25 bar komprimiert, auf 30°C abgekühlt und am untersten Boden der ersten Absorptionskolonne (∅= 8 cm, Höhe= 400 cm, mit Packung Kühni Rombopack) zugeführt. Von oben werden 73 kg/h technisches Nitrobenzol (von der ersten Desorberstufe kommend) zugeführt. Das abgereicherte Abgas am Kopf der ersten Absorptionskolonne wird verworfen. Das beladene Lösungsmittel am Sumpf der ersten Absorptionskolonne wird in einem Flash-Behälter (∅= 20 cm, Höhe= 50 cm bei 30°C betrieben) auf 1 bar entspannt. Das Lösungsmittel wird dann in die erste Absorberstufe zurückgepumpt. Das desorbierte Produktgas aus der ersten Desorberstufe wird dann auf 25 bar komprimiert, auf 30°C abgekühlt und am untersten Boden einer zweiten Absorptionskolonne (∅= 5 cm, Höhe= 400 cm, mit Packung Kühni Rombopack) zugeführt. Von oben werden 16 kg/h technisches Nitrobenzol (von der zweiten Desorberstufe kommend) zugeführt. Das abgereichte Abgas am Kopf der zweiten Absorptionskolonne wird verworfen. Das beladene Lösungsmittel am Sumpf der zweiten Absorptionskolonne wird in einem zweiten Flash-Behälter (∅= 10 cm, Höhe= 40 cm bei 30°C betrieben) auf 1 bar entspannt. Das Lösungsmittel wird zurück in die zweite Absorberstufe gepumpt. Das desorbierte Produktgas aus zweiten Desorberstufe wird dann am untersten Boden einer zweiten Waschkolonne (∅= 0,5 cm, Höhe= 300 cm, mit 10 mm Pallringen) zugeführt. Von oben werden 24 kg/h einer wässrigen NaHCO₃-Lösung (Konzentration: 40 g/kg) zugeführt. Das Gas am Kopf der zweiten Waschkolonne (34,2 mol/h) wird dann auf 25 bar komprimiert auf -30 °C abgekühlt und das flüssige Produkt analysiert. Die Zusammensetzung ist ebenfalls in Tabelle 1.6 angegeben.

**Tabelle 1.6**

| Komponente | Zusammensetzung / Vol.-% | |
|---|---|---|
| | Eduktgas | Flüssiges Produkt |
| N₂O | 22 | 92,9 |
| O₂ | 8,2 | 0,03 |
| N₂ | 42,2 | 0,6 |
| NO₂ | 22,1 | 0,08 |
| NO | 1 | 0 |
| CO₂ | 2,3 | 4,9 |

Die formale Ausbeute an N₂O (definiert als 100×( mol N₂O im verflüssigten Gas)/(mol N₂O im Eduktgas der 1. Stufe)) beträgt 69 %.

### Beispiel 7 :

Aus entsprechenden Vorlagebehältem werden 2000 g/h cis,trans,trans-1,5,9-Cyclododecatrien (davon 200 g/h frisch und 1800 g/h rückgeführtes Edukt) und 73 g/h eines flüssigen Gemisches mit 93,5 Gew.-% N₂O über einen statischen Mischer in einen Rohrreaktor (Doppelmantelrohr, gewickelt, Innendurchmesser 6 mm, Länge 36 m) gepumpt. Das Rohr wird durch Umpumpen von Wärmeträgeröl im Mantel auf 280°C thermostatisiert. Der Reaktorinnendruck wird auf 100 bar geregelt. Nach Passieren der Reaktionszone wird das Reaktionsgemisch in zwei Flashbehältern zunächst auf 3 bar und anschließend auf 60 mbar entspannt, um den gebildeten N₂, unumgesetztes N₂O und im N₂O enthaltenen Inerte (hauptsächlich N₂ und CO₂) abzuführen.

Das flüssige Produkt wird dann in einer Kolonne mit mindestens 7 theoretischen Trennstufen bei 60 mbar destilliert (T_{Sumpf}=170°C, T_{Kopf}=130°C). Als Kopfprodukt erhält man im Mittel 1800 g/h unumgesetztes cis,trans,trans-1,5,9-Cyclododecatrien, das wieder in die Reaktion zurückgeführt wird. Der Sumpfaustrag, der hauptsächlich Cyclododeca-4,8-dienon enthält, wird in eine weitere Kolonne mit mindestens 12 theoretischen Trennstufen überführt und bei 45 mbar destilliert. Hier wird das Cyclododeca-4,8-dienon als Isomerengemisch über Kopf abdestilliert (T_{Sumpf}=193°C, T_{Kopf}=160°C).

Es werden im Mittel 209 g/h Cyclododeca-4,8-dienon erhalten. Die Selektivität zu Cyclo-dodeca-4,8-dienon beträgt 95% (bezogen auf umgesetztes cis,trans,trans-1,5,9-Cyclododecatrien).

## Patentansprüche

1. Verfahren zur Reinigung und Aufkonzentrierung eines Gasgemisches enthaltend Distickstoffmonoxid, mindestens umfassend die folgenden Schritte:
A1 Absorption des Gasgemisches in einem organischen Lösungsmittel
A2 Desorption des Gasgemisches aus dem beladenen organischen Lösungsmittel
B Einstellen des Gehalts an Stickoxiden NOₓ in dem Gasgemisch auf höchstens 0,5 Vol.-%, bezogen auf das Gesamtvolumen des Gasgemisches,
wobei das organische Lösungsmittel ausgewählt ist aus der Gruppe bestehend aus Toluol, Nitrobenzol, 1,2-Dichlorbenzol, Tetradecan und Phthalsäuredimethylester.

2. Verfahren zur Reinigung und Aufkonzentrierung eines Gasgemisches enthaltend Distickstoffmonoxid, mindestens umfassend die folgenden Schritte:
A1 Absorption des Gasgemisches in einem organischen Lösungsmittel
A2 Desorption des Gasgemisches aus dem beladenen organischen Lösungsmittel
B Einstellen des Gehalts an Stickoxiden NOₓ in dem Gasgemisch auf höchstens 0,5 Vol.-%, bezogen auf das Gesamtvolumen des Gasgemisches,
wobei die Schritte A1 und A2 in einer Trennwandkolonne durchgeführt werden.

3. Verfahren zur Reinigung und Aufkonzentrierung eines Gasgemisches enthaltend Distickstoffmonoxid, mindestens umfassend die folgenden Schritte:
A1 Absorption des Gasgemisches in einem organischen Lösungsmittel
A2 Desorption des Gasgemisches aus dem beladenen organischen Lösungsmittel
B Einstellen des Gehalts an Stickoxiden NOₓ in dem Gasgemisch auf höchstens 0,5 Vol.-%, bezogen auf das Gesamtvolumen des Gasgemisches,
wobei das Verfahren mehrere Schritte A1 und A2 umfasst.

4. Verfahren zur Reinigung und Aufkonzentrierung eines Gasgemisches enthaltend Distickstoffmonoxid, mindestens umfassend die folgenden Schritte:
A1 Absorption des Gasgemisches in einem organischen Lösungsmittel
A2 Desorption des Gasgemisches aus dem beladenen organischen Lösungsmittel
B Einstellen des Gehalts an Stickoxiden NOₓ in dem Gasgemisch auf höchstens 0,5 Vol.-%, bezogen auf das Gesamtvolumen des Gasgemisches,
wobei Schritt B die Absorption von Stickoxiden in saurer oder alkalischer Lösung umfasst.

5. Verfahren zur Reinigung und Aufkonzentrierung eines Gasgemisches enthaltend Distickstoffmonoxid, mindestens umfassend die folgenden Schritte:
A1 Absorption des Gasgemisches in einem organischen Lösungsmittel
A2 Desorption des Gasgemisches aus dem beladenen organischen Lösungsmittel
B Einstellen des Gehalts an Stickoxiden NOₓ in dem Gasgemisch auf höchstens 0,5 Vol.-%, bezogen auf das Gesamtvolumen des Gasgemisches,
wobei das erhaltene Gasgemisch verflüssigt wird.

6. Verfahren nach einem der vorherigen Ansprüche 1 bis 5, **dadurch gekennzeichnet, dass** das Gasgemisch enthaltend Distickstoffmonoxid das Abgas einer Adipinsäureanlage und/oder einer Dodecandisäureanlage und/oder einer Hydroxylaminanlage und/oder einer mit dem Abgas einer Adipinsäureanlage und/oder einer Dodecandisäureanlage und/oder einer Hydroxylaminanlage betriebenen Salpetersäureanlage ist.

7. Verfahren nach einem der vorherigen Ansprüche 1 bis 6, **dadurch gekennzeichnet, dass** die Schritte A1 und A2 vor dem Schritt B ausgeführt werden.

8. Verfahren nach einem der Ansprüche 1 bis 6, **dadurch gekennzeichnet, dass** der Schritt B vor den Schritten A1 und A2 ausgeführt wird.

9. Verfahren zur Herstellung eines Ketons, mindestens umfassend die folgenden Schritte
A1 Absorption eines Gasgemisches enthaltend Distickstoffmonoxid in einem organischen Lösungsmittel
A2 Desorption des Gasgemisches aus dem beladenen organischen Lösungsmittel
B Einstellen des Gehalts an Stickoxiden NOₓ in dem Gasgemisch auf höchstens 0,5 Vol.-%, bezogen auf das Gesamtvolumen des Gasgemisches
C Inkontaktbringen des Gasgemisches mit mindestens einem Olefin,
wobei das in Schritt C eingesetzte Gasgemisch verflüssigt ist.

10. Verfahren nach Anspruch 9, **dadurch gekennzeichnet, dass** die Schritte A1 und A2 vor dem Schritt B ausgeführt werden.

11. Verfahren nach Anspruch 9, **dadurch gekennzeichnet, dass** der Schritt B vor den Schritten A1 und A2 ausgeführt wird.

12. Verfahren nach einem der Ansprüche 9 bis 11, **dadurch gekennzeichnet, dass** das Olefin ausgewählt ist aus der Gruppe bestehend aus Cyclopenten, Cyclododecen und 1,5,9-Cyclododecatrien.

## Claims

1. A process for purifying and concentrating a gas mixture comprising dinitrogen monoxide, at least comprising the following steps:
A1 absorbing the gas mixture in an organic solvent
A2 desorbing the gas mixture from the laden organic solvent
B adjusting the content of nitrogen oxides NOₓ in the gas mixture to at most 0.5% by volume based on the total volume of the gas mixture,
the organic solvent being selected from the group consisting of toluene, nitrobenzene, 1,2-dichlorobenzene, tetradecane and dimethyl phthalate.

2. A process for purifying and concentrating a gas mixture comprising dinitrogen monoxide, at least comprising the following steps:
A1 absorbing the gas mixture in an organic solvent
A2 desorbing the gas mixture from the laden orga- nic solvent
B adjusting the content of nitrogen oxides NOₓ in the gas mixture to at most 0.5% by volume based on the total volume of the gas mixture, steps A1 and A2 being carried out in a dividing wall column.

3. A process for purifying and concentrating a gas mixture comprising dinitrogen monoxide, at least comprising the following steps:
A1 absorbing the gas mixture in an organic solvent
A2 desorbing the gas mixture from the laden orga- nic solvent
B adjusting the content of nitrogen oxides NOₓ in the gas mixture to at most 0.5% by volume based on the total volume of the gas mixture,
the process comprising a plurality of steps A1 and A2.

4. A process for purifying and concentrating a gas mixture comprising dinitrogen monoxide, at least comprising the following steps:
A1 absorbing the gas mixture in an organic solvent
A2 desorbing the gas mixture from the laden orga- nic solvent
B adjusting the content of nitrogen oxides NOₓ in the gas mixture to at most 0.5% by volume based on the total volume of the gas mixture,
step B comprising the absorbing nitrogen oxides in acidic or alkaline solution.

5. A process for purifying and concentrating a gas mixture comprising dinitrogen monoxide, at least comprising the following steps:
A1 absorbing the gas mixture in an organic solvent
A2 desorbing the gas mixture from the laden orga- nic solvent
B adjusting the content of nitrogen oxides NOₓ in the gas mixture to at most 0.5% by volume based on the total volume of the gas mixture,
the resulting gas mixture being liquefied.

6. The process according to any of the preceding claims 1 to 5, wherein the gas mixture comprising dinitrogen monoxide is the offgas of an adipic acid plant and/or of a dodecanedioic acid plant and/or of a hydroxylamine plant and/or of a nitric acid plant operated with the offgas of an adipic acid plant and/or of a dodecanedioic acid plant and/or of a hydroxylamine plant.

7. The process according to any of the preceding claims 1 to 6, wherein steps A1 and A2 are performed before step B.

8. The process according to any of claims 1 to 6, wherein step B is performed before steps A1 and A2.

9. A process for preparing a ketone, at least comprising the following steps
A1 absorbing a gas mixture comprising dinitrogen monoxide in an organic solvent
A2 desorbing the gas mixture from the laden orga- nic solvent
B adjusting the content of nitrogen oxides NOₓ in the gas mixture to at most 0.5% by volume based on the total volume of the gas mixture
C contacting the gas mixture with at least one olefin,
the gas mixture used in step C having been liquefied.

10. The process according to claim 9, wherein steps A1 and A2 are performed before step B.

11. The process according to claim 9, wherein step B is performed before steps A1 and A2.

12. The process according to any of claims 9 to 11, wherein the olefin is selected from the group consisting of cyclopentene, cyclododecene and 1,5,9-cyclododecatriene.

## Revendications

1. Procédé de purification et de concentration d'un mélange gazeux contenant du monoxyde de diazote, comprenant au moins les étapes suivantes :
A1 l'absorption du mélange gazeux dans un solvant organique
A2 la désorption du mélange gazeux du solvant organique chargé
B l'ajustement de la teneur en oxydes d'azote NOₓ dans le mélange gazeux au plus à 0,5 % en volume, par rapport au volume total du mélange gazeux,
le solvant organique étant choisi dans le groupe constitué par le toluène, le nitrobenzène, le 1,2-dichlorobenzène, le tétradécane et l'ester diméthylique de l'acide phtalique.

2. Procédé de purification et de concentration d'un mélange gazeux contenant du monoxyde de diazote, comprenant au moins les étapes suivantes :
A1 l'absorption du mélange gazeux dans un solvant organique
A2 la désorption du mélange gazeux du solvant organique chargé
B l'ajustement de la teneur en oxydes d'azote NOₓ dans le mélange gazeux au plus à 0,5 % en volume, par rapport au volume total du mélange gazeux,
les étapes A1 et A2 étant réalisées dans une colonne à paroi de séparation.

3. Procédé de purification et de concentration d'un mélange gazeux contenant du monoxyde de diazote, comprenant au moins les étapes suivantes :
A1 l'absorption du mélange gazeux dans un solvant organique
A2 la désorption du mélange gazeux du solvant organique chargé
B l'ajustement de la teneur en oxydes d'azote NOₓ dans le mélange gazeux au plus à 0,5 % en volume, par rapport au volume total du mélange gazeux,
le procédé comprenant plusieurs étapes A1 et A2.

4. Procédé de purification et de concentration d'un mélange gazeux contenant du monoxyde de diazote, comprenant au moins les étapes suivantes :
A1 l'absorption du mélange gazeux dans un solvant organique
A2 la désorption du mélange gazeux du solvant organique chargé
B l'ajustement de la teneur en oxydes d'azote NOₓ dans le mélange gazeux au plus à 0,5 % en volume, par rapport au volume total du mélange gazeux,
l'étape B comprenant l'absorption d'oxydes d'azote dans une solution acide ou alcaline.

5. Procédé de purification et de concentration d'un mélange gazeux contenant du monoxyde de diazote, comprenant au moins les étapes suivantes :
A1 l'absorption du mélange gazeux dans un solvant organique
A2 la désorption du mélange gazeux du solvant organique chargé
B l'ajustement de la teneur en oxydes d'azote NOₓ dans le mélange gazeux au plus à 0,5 % en volume, par rapport au volume total du mélange gazeux,
le mélange gazeux obtenu étant liquéfié.

6. Procédé selon l'une quelconque des revendications 1 à 5 précédentes, **caractérisé en ce que** le mélange gazeux contenant du monoxyde de diazote est le gaz d'échappement d'une installation de production d'acide adipique et/ou d'une installation de production de diacide dodécanoïque et/ou d'une installation de production d'hydroxylamine et/ou d'une installation de production d'acide nitrique exploitée avec le gaz d'échappement d'une installation de production d'acide adipique et/ou d'une installation de production de diacide dodécanoïque et/ou d'une installation de production d'hydroxylamine.

7. Procédé selon l'une quelconque des revendications 1 à 6 précédentes, **caractérisé en ce que** les étapes A1 et A2 sont réalisées avant l'étape B.

8. Procédé selon l'une quelconque des revendications 1 à 6, **caractérisé en ce que** l'étape B est réalisée avant les étapes A1 et A2.

9. Procédé de fabrication d'une cétone, comprenant au moins les étapes suivantes
A1 l'absorption d'un mélange gazeux contenant du monoxyde de diazote dans un solvant organique A2 la désorption du mélange gazeux du solvant organique chargé
B l'ajustement de la teneur en oxydes d'azote NOₓ dans le mélange gazeux au plus à 0,5 % en volume, par rapport au volume total du mélange gazeux
C la mise en contact du mélange gazeux avec au moins une oléfine,
le mélange gazeux utilisé à l'étape C étant liquéfié.

10. Procédé selon la revendication 9, **caractérisé en ce que** les étapes A1 et A2 sont réalisées avant l'étape B.

11. Procédé selon la revendication 9, **caractérisé en ce que** l'étape B est réalisée avant les étapes A1 et A2.

12. Procédé selon l'une quelconque des revendications 9 à 11, **caractérisé en ce que** l'oléfine est choisie dans le groupe constitué par le cyclopentène, le cyclododécène et le 1,5,9-cyclododécatriène.
